# EUROPEAN PATENT APPLICATION

(11) **EP 4 464 329 A1**
(43) Date of publication of application: **20.11.2024**
(21) Application number: 23737446.7
(22) Date of filing: 06.01.2023
(51) Int. Cl.: A61K 38/00, A61K 38/26, A61K 38/20, A61K 39/395, A61P 25/28

(54) **PHARMACEUTICAL COMPOSITION FOR PREVENTING OR TREATING DEGENERATIVE BRAIN DISEASES, CONTAINING GLUCAGON-LIKE PEPTIDE-1 AND INTERLEUKIN-1 RECEPTOR ANTAGONIST**

(30) Priority: 10.01.2022 KR 20220003278
(71) Applicant: GI Innovation, Inc., Seoul 05855 (KR)
(72) Inventor: JANG, Myung Ho, Seoul 05855 (KR); CHO, Young-Gyu, Seoul 05855 (KR); OH, Young Min, Seoul 05855 (KR); KIM, Jiyoung, Seoul 05855 (KR)
(74) Representative: HGF
(86) International application number: PCT/KR2023/000320
(87) International publication number: WO 2023/132698

(57) **Abstract**

The present invention relates to a pharmaceutical composition for preventing or treating degenerative brain diseases by using glucagon-like peptide-1 (GLP-1) and an interleukin-1 receptor antagonist (IL-1Ra). A composition comprising a fusion protein including GLP-1 and IL-1Ra, according to the present invention, exhibits the effects of inhibiting the production of amyloid beta, which is known as a material causing Alzheimer's disease, and improving memory and cognitive function. In addition, it has been identified that when a bispecific antibody comprising GLP-1 and an anti-IL-1 antibody or a fusion protein in which II,-1Ra and Fc are linked is administered, amyloid beta plaque deposition in the brain is decreased and a neurogenesis promotion effect is exhibited in Alzheimer's disease model mice. Moreover, it has been identified that when GLP-1 and II,-1Ra are also co-administered, amyloid beta plaque deposition in the brain is decreased in Alzheimer's disease model mice. Therefore, the composition comprising GLP-1 and IL-1R, according to the present invention, can be effectively used in the prevention or treatment of degenerative brain diseases, such as Alzheimer's disease, Parkinson's disease and Huntington's disease, and the alleviation of cognitive impairment.

## Description

### Technical Field

The present invention relates to a pharmaceutical composition for preventing or treating degenerative brain diseases by using glucagon-like peptide-1 (GLP-1) and an interleukin-1 receptor antagonist (IL-1Ra). More specifically, the present invention relates to a composition for treating degenerative brain diseases, comprising a fusion protein comprising glucagon-like peptide-1 and an interleukin-1 receptor antagonist; a bispecific antibody comprising GLP-1 and an anti-IL-1 antibody; a fusion protein in which II,-1Ra and Fc are linked; or an IL-1 receptor antagonist and GLP-1.

### Background Art

Degenerative brain disease is a disease in which degenerative changes occur in nerve cells of the central nervous system, causing various symptoms such as impairment of motor and sensory functions and inhibition of higher-order causal functions such as memory, learning, and computational reasoning. Representative diseases include Alzheimer's disease, Parkinson's disease, and Huntington's disease.

The pathogenesis and cause of Alzheimer's disease are not exactly known. It is known that the core mechanism of the disease is that a small protein called beta amyloid is excessively produced and deposited in the brain, causing harmful effects on brain cells. In addition, hyperphosphorylation, inflammatory response, oxidative damage, and the like of tau protein, which plays an important role in maintaining the skeleton of brain cells, also appear to contribute to brain cell damage and affect the development of the disease. Neurogenic plaques (or senile plaques), a representative pathological finding of the brain, are associated with deposition of beta amyloid protein, and neurofibrillary tangles are associated with tau protein hyperphosphorylation.

It has been reported that approximately 40-50% of all cases of Alzheimer's disease are caused by genetic factors, and people who have immediate family members who have suffered from this disease have a higher risk of developing it than those who do not. A representative risk gene that increases the risk of developing disease includes ApoE ε4 (apolipoprotein E ε4). In addition, it is known that Alzheimer's disease occurs in familial cases when there is a mutation in genes such as amyloid precursor protein (APP), presenilin 1 (PS1), and presenilin 2 (PS2). However, these are only involved in the development of early-onset (presenile) Alzheimer's disease, which occurs in people in their 40s and 50s, and are unrelated to the onset of most late-onset (senile) Alzheimer's diseases.

Pathological characteristics of Alzheimer's disease include senile plaques accumulating on the outside of nerve cells, neurofibrillary tangles that look like tangled threads within the cell body of nerve cells, neuronal loss of nerve cells, and the like. These pathological characteristics are present in all cases of Alzheimer's disease. Among these, the main component of senile plaque is aggregated amyloid beta peptide (Aβ), and it is known that necrosis of brain nerve cells caused by amyloid beta peptide leads to the development of Alzheimer's disease (Reisa Sperling et.al., Neuron, 84(3):608-622, 2014).

Tacrine, rivastigmine, galantamine, donepezil, and memantine have been approved by the FDA as therapeutic agents for Alzheimer's disease, but most of the currently used therapeutic agents for Alzheimer's disease are merely neurodegeneration-alleviating substances that alleviate symptoms. Most of these are anti-inflammatory drugs and have side effects such as hepatotoxicity and damage to the mucous membrane of the digestive tract. In addition, the above therapeutic agents have limitations in that they are limited to alleviating symptoms rather than curing them. Therefore, there is a need to discover effective therapeutic agents for degenerative brain diseases.

### Detailed Description of Invention

### Technical Problem

Accordingly, the present inventors conducted research to effectively treat degenerative brain diseases, in particular Alzheimer's disease. As a result, it has been identified that a fusion protein (GLP-1-Fc-II,-1Ra) comprising glucagon-like peptide-1 (GLP-1) and an interleukin-1 receptor antagonist (IL-1Ra) suppressed amyloid beta plaque deposition in the brain of an animal model of Alzheimer's disease and also showed significant effects in cognitive function and behavioral tests. In addition, it has been identified that when a bispecific antibody (GLP-1-anti-IL-1 antibody) comprising GLP-1 and an anti-IL-1 antibody or a fusion protein (Fc-IL-1Ra) in which II,-1Ra and Fc are linked is administered, amyloid beta plaque deposition in the brain is decreased and a neurogenesis promotion effect is exhibited in Alzheimer's disease model mice. In addition, it has been identified that when GLP-1 and II,-1Ra are also co-administered, amyloid beta plaque deposition in the brain is decreased in Alzheimer's disease model mice. Accordingly, the present inventors revealed that a composition comprising GLP-1 and II,-1Ra can be used as a therapeutic agent for degenerative brain diseases, thereby completing the present invention.

### Solution to Problem

In order to achieve the above object, in one aspect of the present invention, there is provided a pharmaceutical composition for preventing or treating degenerative brain diseases, comprising GLP-1 or a variant thereof, and an IL-1 receptor antagonist or a fragment thereof as active ingredients.

In another aspect of the present invention, there is provided a bispecific antibody in which GLP-1 or a variant thereof is linked to an anti-IL-1 antibody or a fragment thereof.

In another aspect of the present invention, there is provided a pharmaceutical composition for preventing or treating degenerative brain diseases, comprising the bispecific antibody as an active ingredient.

In another aspect of the present invention, there is provided a fusion protein comprising an IL-1 receptor antagonist or a fragment thereof and an Fc region fragment or a variant thereof.

In another aspect of the present invention, there is provided a fusion protein dimer in which the two fusion proteins are linked.

In another aspect of the present invention, there is provided a pharmaceutical composition for preventing or treating degenerative brain diseases, comprising the fusion protein or the fusion protein dimer as an active ingredient.

### Effects of Invention

A fusion protein (GLP-1-Fc-II,-1Ra) comprising glucagon-like peptide-1 (GLP-1) and an interleukin-1 receptor antagonist (II,-1Ra), according to the present invention, exhibits the effects of inhibiting the production of amyloid beta, which is known as a material causing Alzheimer's disease, and improving memory and cognitive function. In addition, when a bispecific antibody (GLP-1-anti-IL-1 antibody) comprising GLP-1 and an anti-IL-1 antibody or a fusion protein (Fc-IL-1Ra) in which II,-1Ra and Fc are linked is administered, and when GLP-1 and an IL-1 receptor antagonist are administered in combination, the production of amyloid beta can be inhibited. Therefore, the composition comprising GLP-1 and IL-1R, according to the present invention, can be effectively used in the prevention or treatment of degenerative brain diseases, such as Alzheimer's disease, Parkinson's disease and Huntington's disease, and the alleviation of cognitive impairment.

### Brief Description of Drawings

Figure 1 illustrates the structure of the fusion protein dimer GI210B1 (GLP-1-IgG4 Fc-IL-1Ra) according to one embodiment of the present invention.
Figure 2 illustrates the results obtained by identifying the prepared GI210B1 by SDS-PAGE.
Figure 3 illustrates the results obtained by identifying the prepared GI210B1 by Western blot.
Figure 4 illustrates the structure of the fusion protein dimer GI210B1CN (GLP-1-IgG4 Fc) according to one embodiment of the present invention.
Figure 5 illustrates the structure of the fusion protein dimer GI210B1C1 (IgG4 Fc-IL-1Ra) according to one embodiment of the present invention.
Figure 6 illustrates the structure of the bispecific antibody GI212B2 (GLP-1-anti-IL-1 antibody) according to one embodiment of the present invention.
Figure 7 illustrates the results obtained by identifying the prepared GI212B2 by SDS-PAGE.
Figure 8 illustrates the results obtained by identifying the prepared GI212B2 by SE-HPLC.
Figure 9 is a graph showing the results obtained by measuring the binding ability of GI210B1 to IL-IRα (interleukin-1 receptor alpha).
Figure 10 illustrates an experimental schedule to confirm the therapeutic efficacy of GI210B1 for Alzheimer's disease.

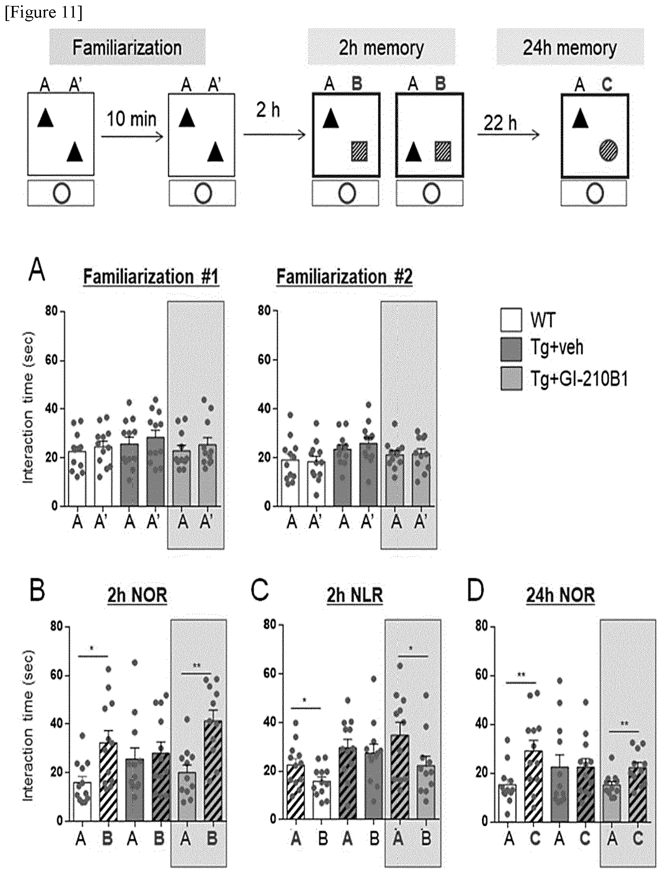

Figure 11 illustrates the results obtained by performing a novel object recognition test in the wild type mice (WT), the Tg-APP/PS1 mice administered with saline (Tg+Veh), and the Tg-APP/PS1 mice administered with GI210B1 (Tg+GI210B1). (A) is a graph showing the results obtained by measuring the time taken for the mouse to recognize two objects during the familiarization stage; (B) is a graph showing the results obtained by measuring the time taken for the mouse to recognize two objects in a 2-hour-NOR test; (C) is a graph showing the results obtained by measuring the time taken for the mouse to recognize two objects in a 2-hour-NLR test; and (D) is a graph showing the results obtained by measuring the time taken for the mouse to recognize two objects in a 4-hour-NOR test (*p<0.05; **p<0.01).

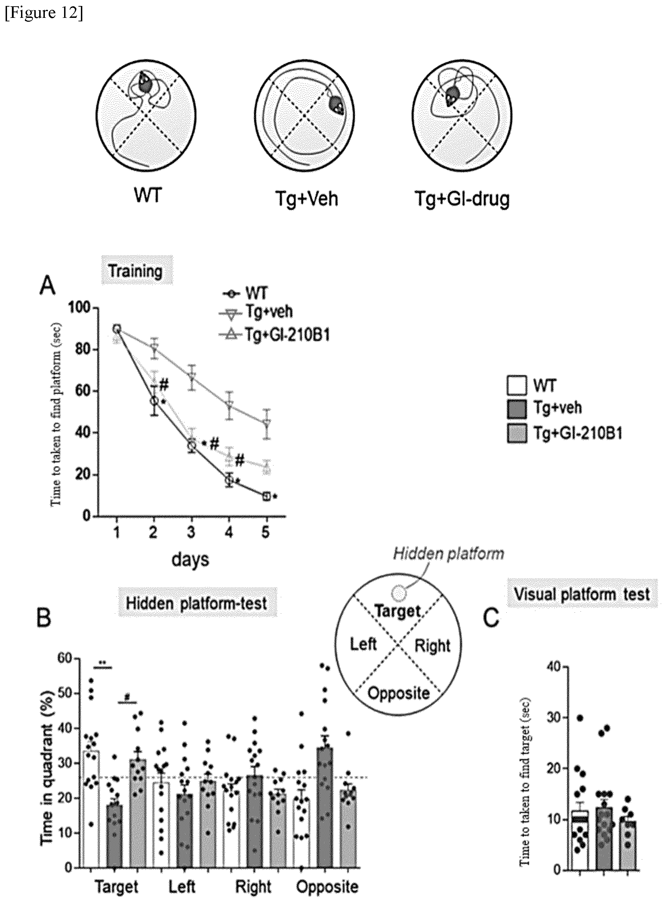

Figure 12 illustrates the results obtained by performing a water maze test in the wild type mice (WT), the Tg-APP/PS1 mice administered with saline (Tg+Veh), and the Tg-APP/PS1 mice administered with GI210B1 (Tg+GI210B1). (A) is a graph showing the results obtained by measuring hidden platform spatial perception training; (B) is a graph showing the results obtained by measuring the time taken to recognize the target quadrant in a hidden platform test; and (C) is a graph showing the results obtained by measuring the time taken to search the target in a visual platform test (*p<0.05; **p<0.01; #p<0.05).
Figure 13 illustrates the results obtained by performing a passive avoidance test in the wild type mice (WT), the Tg-APP/PS1 mice administered with saline (Tg+Veh), and the Tg-APP/PS1 mice administered with GI210B1 (Tg+GI210B1). (A) is a graph showing the results obtained by measuring the time taken for the mouse to move to a dark chamber 24 hours, 72 hours, or 120 hours after the shock was applied (post-shock); and (B) is a graph showing the results obtained by measuring the freezing time of the mouse in a light chamber 24 hours after the shock was applied (post-shock) (*p<0.05).
Figure 14 is a graph showing the results obtained by calculating the number of plaques (A) and the plaque area (B) per unit area of brain tissues (parietal cortex, hippocampus, and piriform cortex) in the Tg-APP/PS1 mice (Tg) and the Tg-APP/PS1 mice administered with GI210B1 (Tg+GI210B1) (*p<0.05).
Figure 15 illustrates the results obtained by staining Aβ plaques in brain tissues (parietal cortex, hippocampus, and piriform cortex) of the Tg-APP/PS1 mice (Tg) and the Tg-APP/PS1 mice administered with GI210B1 (Tg+GI210B1) using thioflavin S.
Figure 16 is a schematic diagram showing the CA1 region of the hippocampus.
Figure 17 illustrates the results obtained by fluorescently staining the CA1 region of the hippocampus of the wild type mice (WT), the Tg-APP/PS1 mice (Tg), and the Tg-APP/PS1 mice administered with GI210B1 (Tg+GI210B1) using MAP-2 (microtubule associated protein 2).
Figure 18 is a graph showing the results obtained by measuring MAP-2 expression in the CA1 region of the hippocampus of the wild type mice (WT), the Tg-APP/PS1 mice (Tg), and the Tg-APP/PS1 mice administered with GI210B1 (Tg+GI210B1) (*p<0.05; **p<0.01).
Figure 19 illustrates the results obtained by staining the DG (dentate gyrus) region of the hippocampus of the wild type mice (WT), the Tg-APP/PS1 mice (Tg), and the Tg-APP/PS1 mice administered with GI210B1 (Tg+GI210B1) using DCX (doublecortin).
Figure 20 is a graph showing the results obtained by measuring the number of DCX positive cells in the DG region of the hippocampus of the wild type mice (WT), the Tg-APP/PS1 mice (Tg), and the Tg-APP/PS1 mice administered with GI210B1 (Tg+GI210B1) (**p<0.01).
Figure 21 illustrates the results obtained by staining the DG region of the hippocampus of the wild type mice (WT), the Tg-APP/PS1 mice (Tg), and the Tg-APP/PS1 mice administered with GI210B 1 (Tg+GI210B 1) using Ki-67.
Figure 22 is a graph showing the results obtained by measuring the number of Ki-67 positive cells in the DG region of the hippocampus of the wild type mice (WT), the Tg-APP/PS1 mice (Tg), and the Tg-APP/PS1 mice administered with GI210B1 (Tg+GI210B1) (*p<0.05).
Figure 23 illustrates an experimental schedule to confirm the therapeutic efficacy of GI210B1, GI210B1C1, and GI210B1CN for Alzheimer's disease.
Figure 24 illustrates the results obtained by staining Aβ plaques in brain tissues (parietal cortex, hippocampus, and piriform cortex) of the Tg-APP/PS1 mice administered with PBS (Tg+Veh), the Tg-APP/PS1 mice administered with GI210B1 (Tg+GI210B 1), the Tg-APP/PS1 mice administered with GI210B1C1 (Tg+GI210B1C1), and the Tg-APP/PS1 mice administered with GI210B1CN (Tg+GI210B1CN) using thioflavin S.
Figure 25 is a graph showing the results obtained by calculating the number of plaques per unit area (A) and the plaque area per unit area (B) of brain tissues (parietal cortex, hippocampus, and piriform cortex) in the Tg-APP/PS1 mice administered with PBS (Tg+Veh), the Tg-APP/PS1 mice administered with GI210B1 (Tg+GI210B1), the Tg-APP/PS1 mice administered with GI210B1C1 (Tg+GI210B1C1), and the Tg-APP/PS1 mice administered with GI210B1CN (Tg+GI210B1CN) (*p<0.05; **p<0.01).
Figure 26 illustrates the results obtained by fluorescently staining the CA1 region of the hippocampus of the wild type mice administered with PBS (WT+Veh), the Tg-APP/PS1 mice administered with PBS (Tg+Veh), the Tg-APP/PS1 mice administered with GI210B1 (Tg+GI210B1), the Tg-APP/PS1 mice administered with GI210B1C1 (Tg+GI210B1C1), and the Tg-APP/PS1 mice administered with GI210B1CN (Tg+GI210B1CN) using MAP2 (microtubule associated protein 2).
Figure 27 is a graph showing the results obtained by identifying the level of MAP2 expression in the CA1 region of the hippocampus of the wild type mice administered with PBS (WT+Veh), the Tg-APP/PS1 mice administered with PBS (Tg+Veh), the Tg-APP/PS1 mice administered with GI210B1 (Tg+GI210B1), the Tg-APP/PS1 mice administered with GI210B1C1 (Tg+GI210B1C1), and the Tg-APP/PS1 mice administered with GI210B1CN (Tg+GI210B1CN).
Figure 28 illustrates the results obtained by staining the DG region of the hippocampus of the wild type mice administered with PBS (WT+Veh), the Tg-APP/PS1 mice administered with PBS (Tg+Veh), the Tg-APP/PS1 mice administered with GI210B1 (Tg+GI210B1), the Tg-APP/PS1 mice administered with GI210B1C1 (Tg+GI210B1C1), and the Tg-APP/PS1 mice administered with GI210B1CN (Tg+GI210B1CN) using DCX (doublecortin).
Figure 29 is a graph showing the DCX positive cells in the DG region of the hippocampus of the wild type mice administered with PBS (WT+Veh), the Tg-APP/PS1 mice administered with PBS (Tg+Veh), the Tg-APP/PS1 mice administered with GI210B1 (Tg+GI210B1), the Tg-APP/PS1 mice administered with GI210B1C1 (Tg+GI210B1C1), and the Tg-APP/PS1 mice administered with GI210B1CN (Tg+GI210B1CN) (**p<0.01 vs WT+Veh; #p<0.05 vs TG+Veh).
Figure 30 is a schematic diagram showing an experiment to confirm the preventive and therapeutic efficacy of administration of an interleukin-1 receptor antagonist and GLP-1 in combination for Alzheimer's disease.

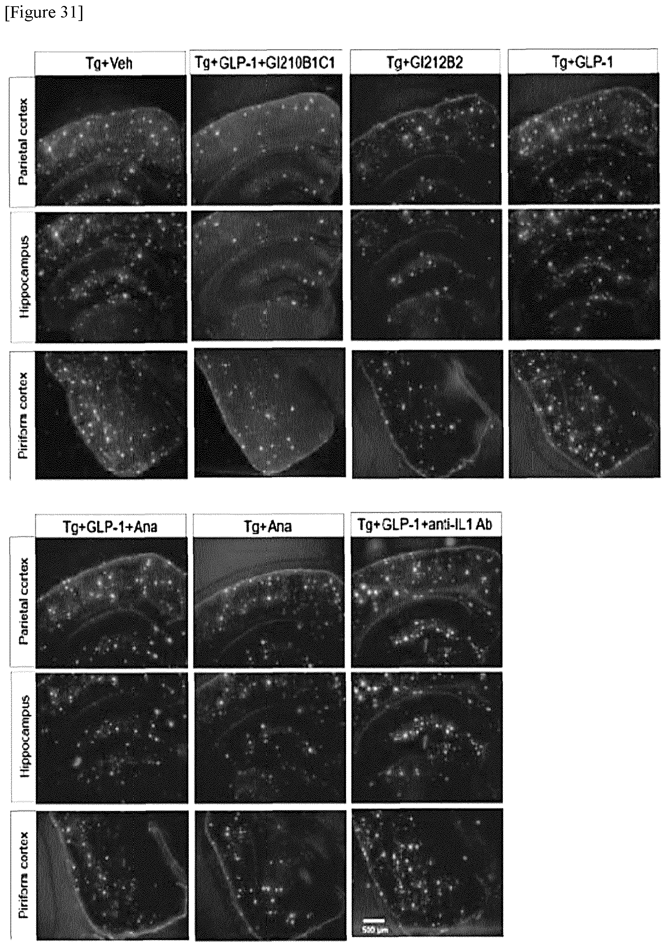

Figure 31 illustrates the results obtained by staining Aβ plaques in brain tissues (parietal cortex, hippocampus, and piriform cortex) of the Tg-APP/PS1 mice administered with PBS (Tg+Veh), the Tg-APP/PS1 mice administered with GLP-1 and GI210B1C1 in combination (Tg+GLP-1+GI210B1C1), the Tg-APP/PS1 mice administered with GI212B2 (Tg+GI212B2), the Tg-APP/PS1 mice administered with GLP-1 (Tg+GLP-1), the Tg-APP/PS1 mice administered with GLP-1 and anakinra in combination (Tg+GLP-1+Ana), the Tg-APP/PS1 mice administered with anakinra (Tg+Ana), and the Tg-APP/PS1 mice administered with GLP-1 and an anti-IL-1 antibody in combination (Tg+GLP-1+anti-IL-1 antibody) using thioflavin S.

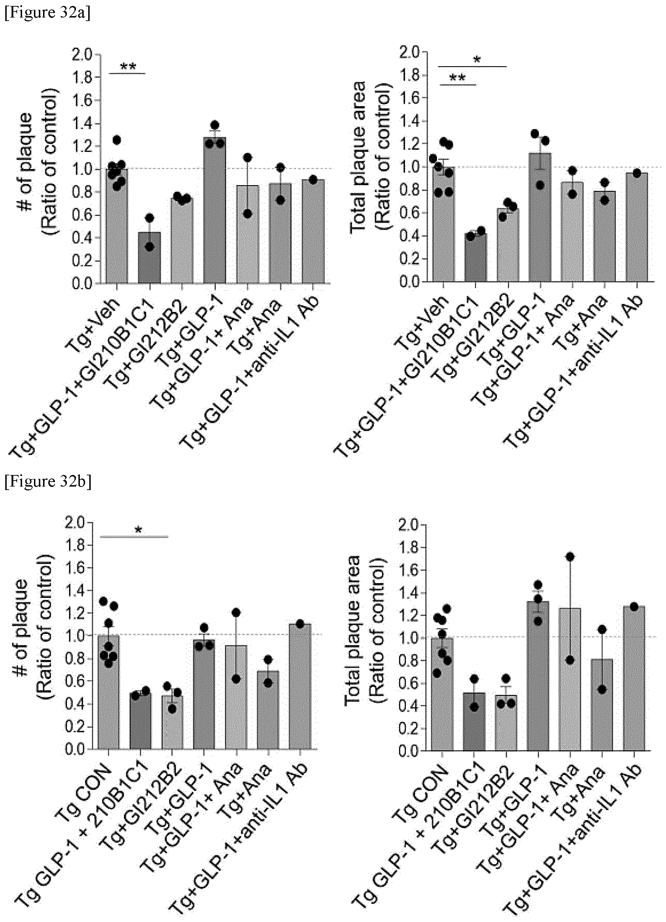

Figure 32a is a graph showing the results obtained by calculating the number of Aβ plaques per unit area in the parietal cortex of the Tg-APP/PS1 mice administered with PBS (Tg+Veh), the Tg-APP/PS1 mice administered with GLP-1 and GI210B1C1 in combination (Tg+GLP-1+GI210B1C1), the Tg-APP/PS1 mice administered with GI212B2 (Tg+GI212B2), the Tg-APP/PS1 mice administered with GLP-1 (Tg+GLP-1), the Tg-APP/PS1 mice administered with GLP-1 and anakinra in combination (Tg+GLP-1+Ana), the Tg-APP/PS1 mice administered with anakinra (Tg+Ana), and the Tg-APP/PS1 mice administered with GLP-1 and an anti-IL-1 antibody in combination (Tg+GLP-1+anti-IL-1 antibody) (*p<0.05; **p<0.01).
Figure 32b is a graph showing the results obtained by calculating the number of Aβ plaques per unit area in the hippocampus of the Tg-APP/PS1 mice administered with PBS (Tg+Veh), the Tg-APP/PS1 mice administered with GLP-1 and GI210B1C1 in combination (Tg+GLP-1+GI210B1C1), the Tg-APP/PS1 mice administered with GI212B2 (Tg+GI212B2), the Tg-APP/PS1 mice administered with GLP-1 (Tg+GLP-1), the Tg-APP/PS1 mice administered with GLP-1 and anakinra in combination (Tg+GLP-1+Ana), the Tg-APP/PS1 mice administered with anakinra (Tg+Ana), and the Tg-APP/PS1 mice administered with GLP-1 and an anti-IL-1 antibody in combination (Tg+GLP-1+anti-II,-1 antibody) (*p<0.05).

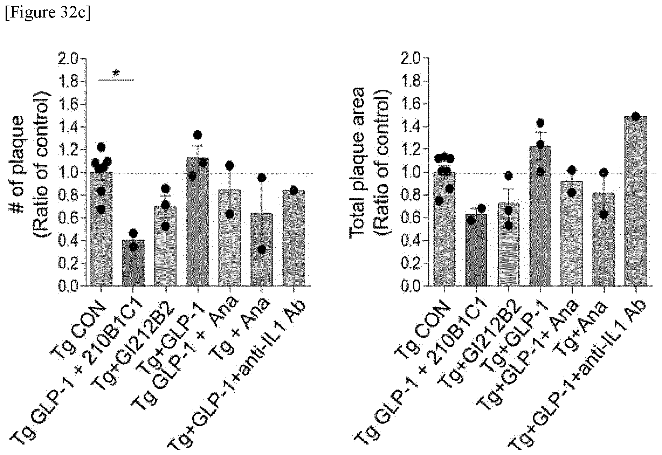

Figure 32c is a graph showing the results obtained by calculating the number of Aβ plaques per unit area in the piriform cortex of the Tg-APP/PS1 mice administered with PBS (Tg+Veh), the Tg-APP/PS1 mice administered with GLP-1 and GI210B1C1 in combination (Tg+GLP-1+GI210B1C1), the Tg-APP/PS1 mice administered with GI212B2 (Tg+GI212B2), the Tg-APP/PS1 mice administered with GLP-1 (Tg+GLP-1), the Tg-APP/PS1 mice administered with GLP-1 and anakinra in combination (Tg+GLP-1+Ana), the Tg-APP/PS1 mice administered with anakinra (Tg+Ana), and the Tg-APP/PS1 mice administered with GLP-1 and an anti-IL-1 antibody in combination (Tg+GLP-1+anti-II,-1 antibody) (*p<0.05).

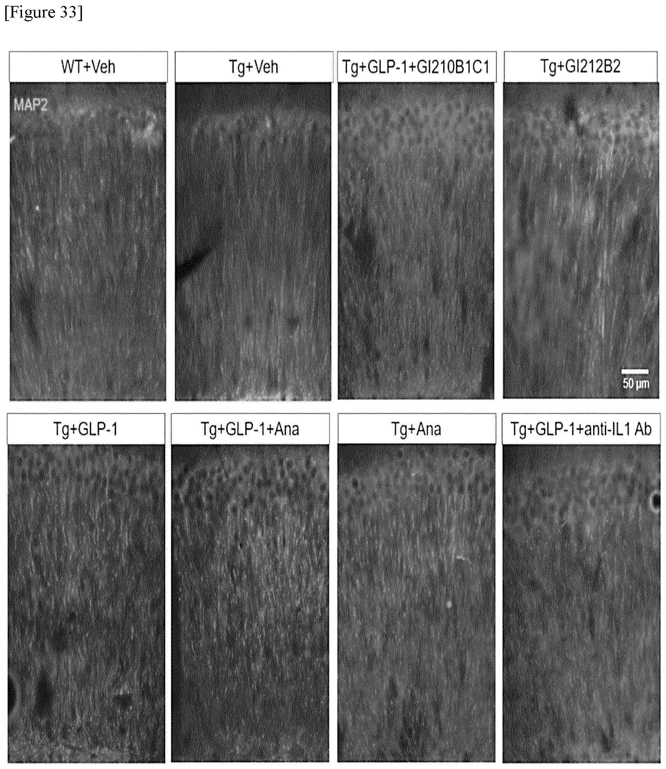

Figure 33 illustrates the results obtained by fluorescently staining the CA1 region of the hippocampus of the wild type mice administered with PBS (WT+Veh), the Tg-APP/PS1 mice administered with PBS (Tg+Veh), the Tg-APP/PS1 mice administered with GLP-1 and GI210B1C1 in combination (Tg+GLP-1+GI210B1C1), the Tg-APP/PS1 mice administered with GI212B2 (Tg+GI212B2), the Tg-APP/PS1 mice administered with GLP-1 (Tg+GLP-1), the Tg-APP/PS1 mice administered with GLP-1 and anakinra in combination (Tg+GLP-1+Ana), the Tg-APP/PS1 mice administered with anakinra (Tg+Ana), and the Tg-APP/PS1 mice administered with GLP-1 and an anti-IL-1 antibody in combination (Tg+GLP-1+anti-IL-1 antibody) using MAP2.

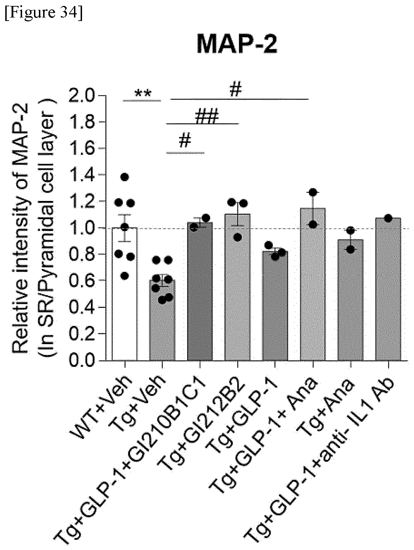

Figure 34 is a graph showing the results obtained by identifying the level of MAP2 expression in the CA1 region of the hippocampus of the wild type mice administered with PBS (WT+Veh), the Tg-APP/PS1 mice administered with PBS (Tg+Veh), the Tg-APP/PS1 mice administered with GLP-1 and GI210B1C1 in combination (Tg+GLP-1+GI210B1C1), the Tg-APP/PS1 mice administered with GI212B2 (Tg+GI212B2), the Tg-APP/PS1 mice administered with GLP-1 (Tg+GLP-1), the Tg-APP/PS1 mice administered with GLP-1 and anakinra in combination (Tg+GLP-1+Ana), the Tg-APP/PS1 mice administered with anakinra (Tg+Ana), and the Tg-APP/PS1 mice administered with GLP-1 and an anti-IL-1 antibody in combination (Tg+GLP-1+anti-II,-1 antibody) (*p<0.05 vs WT+Veh; #p<0.05, ##p<0.01 vs TG+Veh).

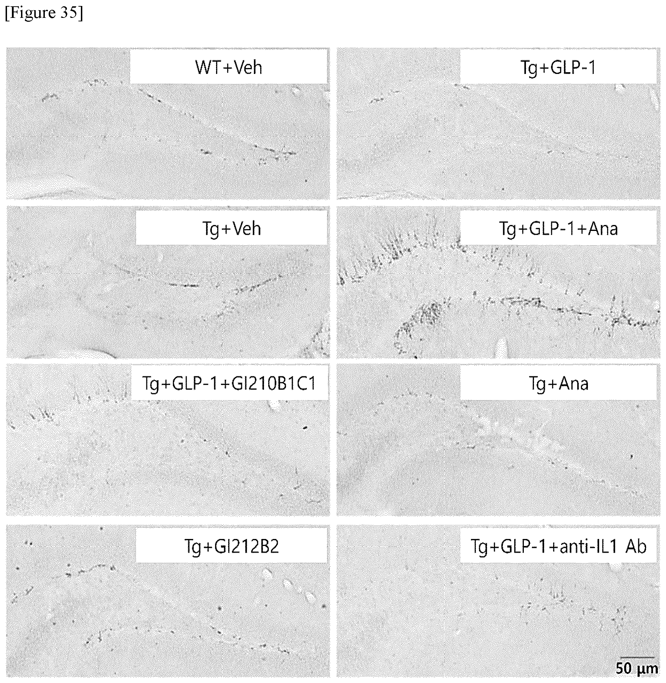

Figure 35 illustrates the results obtained by staining the DG region of the hippocampus of the wild type mice administered with PBS (WT+Veh), the Tg-APP/PS1 mice administered with PBS (Tg+Veh), the Tg-APP/PS1 mice administered with GLP-1 and GI210B1C1 in combination (Tg+GLP-1+GI210B1C1), the Tg-APP/PS1 mice administered with GI212B2 (Tg+GI212B2), the Tg-APP/PS1 mice administered with GLP-1 (Tg+GLP-1), the Tg-APP/PS1 mice administered with GLP-1 and anakinra in combination (Tg+GLP-1+Ana), the Tg-APP/PS1 mice administered with anakinra (Tg+Ana), and the Tg-APP/PS1 mice administered with GLP-1 and an anti-IL-1 antibody in combination (Tg+GLP-1+anti-IL-1 antibody) using DCX.

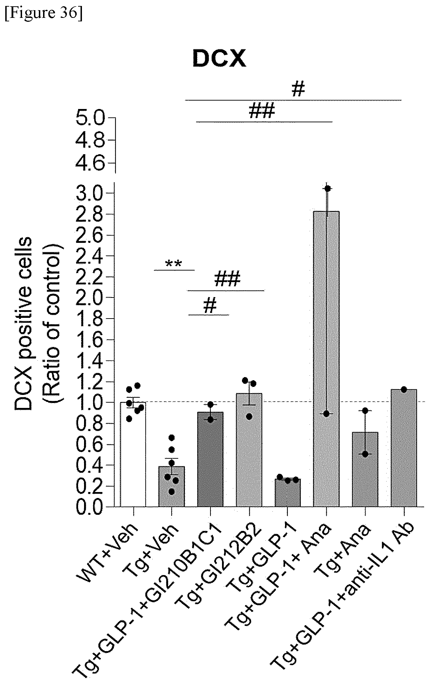

Figure 36 is a graph showing the results obtained by identifying the DCX positive cells in the DG region of the hippocampus of the wild type mice administered with PBS (WT+Veh), the Tg-APP/PS1 mice administered with PBS (Tg+Veh), the Tg-APP/PS1 mice administered with GLP-1 and GI210B1C1 in combination (Tg+GLP-1+GI210B1C1), the Tg-APP/PS1 mice administered with GI212B2 (Tg+GI212B2), the Tg-APP/PS1 mice administered with GLP-1 (Tg+GLP-1), the Tg-APP/PS1 mice administered with GLP-1 and anakinra in combination (Tg+GLP-1+Ana), the Tg-APP/PS1 mice administered with anakinra (Tg+Ana), and the Tg-APP/PS1 mice administered with GLP-1 and an anti-IL-1 antibody in combination (Tg+GLP-1+anti-II,-1 antibody) (**p<0.01 vs WT+Veh; #p<0.05, ##p<0.01 vs TG+Veh).

### Best Mode for Carrying out the Invention

### I. Pharmaceutical composition for preventing or treating degenerative brain diseases, comprising GLP-1 and IL-1 receptor antagonist

In one aspect of the present invention, there is provided a pharmaceutical composition for preventing or treating degenerative brain diseases, comprising GLP-1 or a variant thereof, and an IL-1 receptor antagonist (interleukin-1 receptor antagonist) or a fragment thereof as active ingredients.

As used herein, the term "GLP-1 (glucagon-like peptide-1)" or "glucagon-like peptide-1" is an incretin derived from proglucagon, a prohormone, and is a hormone secreted by intestinal L cells stimulated by nutrients or blood sugar concentration in the intestine.

GLP-1 consists of 30 amino acids and is made from proglucagon. The amino acid sequence of GLP-1 is known to be 100% identical in all mammals. Through post-transcriptional processes, glucagon is produced from proglucagon in pancreatic islet α cells, and GLP-1 is produced in L cells of the ileum and colon. In order to be activated, the produced GLP-1 becomes GLP-1 (7-37) or GLP-1 (7-36) amide form by truncating part of the N terminus. Specifically, the GLP-1 may have the sequence of SEQ ID NO: 1, but is not limited thereto.

In the present specification, GLP-1 or a variant thereof is collectively referred to as the term "GLP-1". GLP-1 and a GLP-1 variant specifically bind to, for example, a GLP-1 receptor (GLP-1R, glucagon-like peptide-1 receptor). The specific binding can be confirmed by a method known to those of ordinary skill in the art.

As used herein, the term "GLP-1 variant" refers to a form in which some amino acids of full-length GLP-1 are substituted, deleted, added, and/or inserted. That is, a GLP-1 variant may have an amino acid sequence different from a wild type GLP-1. However, the GLP-1 variant may have equivalent or similar activity to a wild type GLP-1. Here, "GLP-1 activity" may mean, for example, binding specifically to a GLP-1 receptor, and the specific binding can be measured by a method known to those of ordinary skill in the art.

Specifically, the GLP-1 variant may be one in which some amino acids of a wild type GLP-1 are deleted, modified, substituted, and/or added, and may be prepared by a method known to those of ordinary skill in the art. The GLP-1 variant may comprise at least 5 or more, at least 10 or more, at least 15 or more, or at least 20 or more amino acids of the wild type GLP-1 sequence.

Specifically, the GLP-1 variant may be one in which some amino acids of a wild type GLP-1 are substituted and deleted. One specific example of the GLP-1 variant resulting from amino acid substitution and deletion may be one in which the 2nd and 30th amino acids in the amino acid sequence of SEQ ID NO: 1 are substituted with other amino acids, and one amino acid sequence is added to the C terminus.

In addition, the GLP-1 variant may be one in which some amino acids of a wild type GLP-1 are substituted and added. One specific example of the GLP-1 variant resulting from amino acid substitution and addition may be one in which the 2nd, 10th, 12th, 13th, 14th, 16th, 17th, 19th, 20th, 21th, 24th, 27th, 28th, and 30th amino acids in the amino acid sequence of SEQ ID NO: 1 are substituted with other amino acids, and a 9-amino acid sequence is added to the C terminus. Furthermore, it may be one in which the 2nd and 30th amino acids in the amino acid sequence of SEQ ID NO: 1 are substituted with other amino acids, and one amino acid sequence is added to the C terminus.

At that time, the "amino acid" introduced by the substitution and/or addition may be any one selected from the group consisting of glycine (G), leucine (L), lysine (K), glutamine (Q), methionine (M), glutamic acid (E), valine (V), arginine (R), asparagine (N), proline (P), serine (S), and alanine (A).

In one embodiment, the GLP-1 variant may be one in which A2G and R30G are substituted in the amino acid sequence of SEQ ID NO: 1 and one amino acid sequence G is added to the C terminus. In addition, it may be one in which A2G, V10L, S12K, Y13Q, L14M, G16E, Q17E, A19V, K20R, E21L, A24E, V27K, K28N and R30G are substituted in the amino acid sequence of SEQ ID NO: 1 and a 9-amino acid sequence PSSGAPPPS (SEQ ID NO: 21) is added to the C terminus.

Specifically, the GLP-1 variant may have any one of the amino acid sequences of SEQ ID NOs: 2 to 4.

In addition, the GLP-1 variant may be characterized by an extended in vivo action time. There are many difficulties in developing the wild type GLP-1 into a drug, as it is cleaved and inactivated at a very rapid rate by the DPP-4 (dipeptidyl peptidase-4) enzyme in vivo. Accordingly, the GLP-1 variant is one that has a mutation in a wild type GLP-1 and has reduced cleavage by the DPP-4 enzyme, and may have the characteristic of having a biological half-life that is significantly longer than the biological half-life (1 to 2 minutes) of a wild type GLP-1.

Meanwhile, the wild type GLP-1 is processed in vivo so that the first six amino acids are cleaved from the molecule. Therefore, by convention in the art, the amino terminus of GLP-1 is designated as 7, and the carboxy terminus is designated as 37. According to the above processing, the form of GLP-1 (1-37) without the insulin secretion function can be changed to the active form of GLP-1 (7-37), and it can be further modified in vivo to remove the C-terminal glycine residue and replace it with an amide group, resulting in the form GLP-1 (7-36) amide (the amino acid sequence of SEQ ID NO: 1).

The term "GLP-1" or "glucagon-like peptide-1", unless otherwise stated, refers to any wild type GLP-1 obtained from any vertebrate source, including mammals, for example, primates (e.g., humans) and rodents (e.g., mice and rats). GLP-1 may be obtained from animal cells, and also includes one obtained from recombinant cells capable of producing GLP-1. In addition, GLP-1 may be a wild type GLP-1 or a variant thereof.

### First fusion protein comprising GLP-1 and Fc region and dimer thereof

In the present invention, the GLP-1 or a variant thereof may be a dimer of a first fusion protein further comprising an Fc region or a variant thereof.

At that time, the Fc region of an immunoglobulin refers to a protein that comprises a heavy chain constant region 2 (CH2) and a heavy chain constant region 3 (CH3) of an immunoglobulin, and does not comprise a heavy chain variable region, a heavy chain constant region 1 (CH1) of an immunoglobulin; and a light chain variable region, a light chain constant region 1 (CL1). The immunoglobulin may be IgG, IgA, IgE, IgD, or IgM, and preferably, may be IgG. Specifically, the IgG may be IgG1, IgG2, IgG3, or IgG4, and preferably, may be IgG4. In the present specification, the "Fc region" can be used interchangeably with the "Fc domain".

In addition, the Fc domain of an immunoglobulin may be an Fc domain variant as well as a wild type Fc domain. In addition, as used herein, the term "Fc domain variant" may refer to a form which is different from the wild type Fc domain in terms of glycosylation pattern, has a high glycosylation as compared with the wild type Fc domain, or has a low glycosylation as compared with the wild type Fc domain, or a deglycosylated form. In addition, an aglycosylated Fc domain is included therein. The Fc domain or a variant thereof may be adapted to have an adjusted number of sialic acids, fucosylations, or glycosylations, through culture conditions or genetic manipulation of a host.

In addition, glycosylation of the Fc domain of an immunoglobulin may be modified by conventional methods such as chemical methods, enzymatic methods, and genetic engineering methods using microorganisms. In addition, the Fc domain variant may be in a mixed form of respective Fc domains of immunoglobulin IgG, IgA, IgE, IgD, or IgM. In addition, the Fc domain variant may be in a form in which some amino acids of the Fc domain are substituted with other amino acids. One specific example of the Fc domain variant may have the amino acid sequence of SEQ ID NO: 6.

Specifically, the first fusion protein comprising GLP-1 or a variant thereof, and an Fc region fragment or a variant thereof may consist of the following structural formula (III) or (IV):
N'-[linker (3)]l-Fc region fragment or variant thereof-[linker (4)]m-A-C' (III)
N'-A-[linker (5)]n-Fc region fragment or variant thereof-C' (IV)
wherein, in the structural formulas (III) and (IV),
N' is the N terminus of the fusion protein,
C' is the C terminus of the fusion protein,
A is GLP-1 or a fragment thereof,
the linker (3), linker (4), and linker (5) are peptide linkers, and
l, m, and n are each independently 0 or 1.
At that time, the GLP-1 or a variant thereof is the same as described above.

The peptide linker (3) may consist of 5 to 80 consecutive amino acids, 5 to 50 consecutive amino acids, 5 to 40 consecutive amino acids, 5 to 30 consecutive amino acids, or 5 to 20 amino acids. In one embodiment, the peptide linker (3) may consist of 12 amino acids. In addition, the peptide linker (3) may comprise at least one cysteine. Specifically, it may comprise one, two, or three cysteines. In addition, the peptide linker (3) may be derived from the hinge of an immunoglobulin. In one embodiment, the peptide linker (3) may be a peptide linker consisting of the amino acid sequence of SEQ ID NO: 31.

The peptide linker (4) may consist of 1 to 50 consecutive amino acids, or 3 to 30 consecutive amino acids, or 5 to 20 amino acids. The peptide linker (4) may comprise (G4S)n (where n is an integer of 1 to 10), and may further comprise (G)n. At that time, in (G4S)n and (G)n, n may be each independently 1, 2, 3, 4, 5, 6, 7, 8, 9, or 10. In addition, the linker (4) may be a GS3 linker. In one embodiment, the peptide linker (4) may be a peptide linker consisting of the amino acid sequence of SEQ ID NO: 7. In one embodiment, the peptide linker (4) may comprise the amino acid sequence of SEQ ID NO: 26.

The peptide linker (5) may consist of 5 to 80 consecutive amino acids, 10 to 70 consecutive amino acids, 15 to 60 consecutive amino acids, 20 to 50 consecutive amino acids, or 25 to 40 amino acids. In one embodiment, the peptide linker (5) may consist of 30 amino acids. In addition, the peptide linker (5) may comprise at least one cysteine. Specifically, it may comprise one, two, or three cysteines. In addition, the peptide linker (5) may be derived from the hinge of an immunoglobulin. For example, the hinge may be selected from the hinge regions of various IgG subtype antibodies. In addition, the hinge may be a form in which some amino acids in the hinge region derived from an immunoglobulin are substituted with other amino acids, or may be a sequence in which some amino acid sequences are added. In one embodiment, the peptide linker (5) may be a peptide linker consisting of the amino acid sequence of SEQ ID NO: 5 or SEQ ID NO: 31.

Each amino acid sequence of the first fusion protein is shown in Table 1 below. In addition, one specific example of the first fusion protein (GLP-1-IgG4 Fc, GI210B1CN) may have the amino acid sequence of SEQ ID NO: 22.

**[Table 1]**

| Item | Amino acid sequence | SEQ ID NO |
|---|---|---|
| hGLP-1 | HAEGTFTSDVSSYLEGQAAKEFIAWLVKGR | 1 |
| GLP-1 variant-1 | HGEGTFTSDVSSYLEEQAAKEFIAWLVKGGG | 2 |
| GLP-1 variant-2 | HGEGTFTSDLSKQMEEEAVRLFIEWLKNGGPSSGAPPPS | 3 |
| GLP-1 variant-3 | HAEGTFTSDVSSYLEGQAAKEFIAWLVRGRG | 4 |
| Linker | GGGGSGGGGSGGGGSGGAESKYGPPCPPCP | 5 |
| hIgG4 Fc | APEAAGGPSVFLFPPKPKDQLMISRTPEVTCVVVDVSQEDPEVQFNWYVDGVEVH NAKTKPREEQFNSTYRVVSVLTVLHQDWLNGKEYKCKVSNKGLPSSIEKTISKAK GQPREPQVYTLPPSQEEMTKNQVSLTCLVKGFYPSDIAVEWESNGQPENNYKTTP PVLDSDGSFFLYSRLTVDKSRWQEGNVFSCSVLHEALHNHYTQKSLSLSLG | 6 |
| GI210B1CN (GLP-1-IgG4 Fc) | HGEGTFTSDVSSYLEEQAAKEFIAWLVKGGGGGGGSGGGGSGGGGSGGAESKYGP PCPPCPAPEAAGGPSVFLFPPKPKDQLMISRTPEVTCVVVDVSQEDPEVQFNWYV DGVEVHNAKTKPREEQFNSTYRVVSVLTVLHQDWLNGKEYKCKVSNKGLPSSIEK TISKAKGQPREPQVYTLPPSQEEMTKNQVSLTCLVKGFYPSDIAVEWESNGQPEN | 22 |
| | NYKTTPPVLDSDGSFFLYSRLTVDKSRWQEGNVFSCSVLHEALHNHYTQKSLSLS LG | |

In the present invention, the first fusion protein comprising GLP-1 or a variant thereof, and an Fc region fragment or a variant thereof may exist as a fusion protein dimer in which the two first fusion proteins are linked. At that time, the first fusion protein may be linked by cysteine included in the linker located at the N terminus of the Fc region fragment or a variant thereof. At that time, the linker may comprise the hinge region of an immunoglobulin.

As used herein, the term "interleukin 1 (IL-1)" is one of the members of the interleukin superfamily consisting of 11 cytokines. The interleukin superfamily includes receptors, co-receptors, antagonists, and endogenous antagonists. IL-1α and IL-1β are known to be major agonistic molecules, and an IL-1 receptor antagonist (IL-1Ra) is known to be a representative endogenous antagonist. IL-1α and IL-1β (collectively referred to as IL-1) act as extracellular activators, while II,-1Ra interacts with an IL-1 receptor 1 (IL-1R1) to act as a competitive inhibitor that inhibits the interaction with IL-1R1 of IL-1.

IL-1α and IL-1β are each synthesized as a precursor of 31 kDa. The precursors are each processed by proteolytic enzymes into the mature secreted form of 17 kDa. IL-1β is induced and secreted only by inflammatory signals, and the IL-1β precursor exists in a biologically inactive state until it is processed into the mature secreted form by caspase-1. IL-1β functions as a pro-inflammatory molecule. Conversely, IL-1α exists in a variety of intracellular homeostatic conditions. The IL-1α precursor is processed by the Ca2+ dependent proteolytic enzyme calpain into mature II,-1NTP (N terminus peptide) forms of 17 kDa and 16 kDa.

IL-1α and IL-1β signaling occurs through the IL-1 receptor (IL-1R). IL-1R1 (80 kDa) is a signal transduction receptor, but IL-1R2 (68 kDa) is an induction target and plays a role in suppressing excessive activity of IL-1. When IL-1 binds to IL-1R1, it forms a heterotypic complex with IL-1RAcP (IL-1R accessory protein), which induces the activation of NF-κB and target genes. On the other hand, IL-1R2 lacks the intracellular signaling domain, so even when IL-1 binds to IL-1R2, it is unable to transmit signals into the cell. In addition, IL-1R2 forms a heterotypic complex with IL-1RAcP and inhibits the formation of the heterotypic complex of IL-1R1, and thus competitively inhibits the activity of IL-1R1.

As used herein, the term "IL-1 receptor antagonist (interleukin-1 receptor antagonist)" or "IL-1Ra" refers to an antagonist that inhibits IL-1, which exists in two forms: IL-1α and IL-1β. II,-1Ra is structurally similar to IL-1α and IL-1β, and thus competitively binds to the IL-1 receptor (IL-1R) to inhibit IL-1, an inflammatory cytokine. Therefore, it is known that II,-1Ra can suppress diseases caused by IL-1. In particular, the II,-1Ra is known to play an important pathophysiological role in type 2 diabetes mellitus and atherosclerosis. In the present invention, the II,-1Ra may have the amino acid sequence of SEQ ID NO: 8, but is not limited thereto.

In the present specification, an IL-1 receptor antagonist (IL-1Ra) or a fragment thereof is collectively referred to as the term "IL-1Ra". IL-1Ra and an II,-1Ra fragment specifically bind to, for example, an IL-1 receptor (IL-1R, interleukin-1 receptor). The specific binding can be confirmed by a method known to those of ordinary skill in the art.

As used herein, the term "II,-1Ra fragment" refers to a truncated form of IL-1Ra, and may include a fragment in which a part of the N terminus or C terminus of a full-length II,-1Ra is deleted (truncated). In addition, the II,-1Ra fragment can be used in the same context as the "II,-1Ra variant", which refers to a form in which some amino acids of a full-length II,-1Ra are substituted, deleted, added, and/or inserted. That is, an II,-1Ra variant may have an amino acid sequence different from a wild type IL-1Ra. However, the II,-1Ra variant may have equivalent or similar activity to a wild type IL-1Ra. Here, "II,-1Ra activity" may mean, for example, binding specifically to an IL-1 receptor, and the specific binding can be measured by a method known to those of ordinary skill in the art.

Specifically, the II,-1Ra variant may be one in which some amino acids of a wild type II,-1Ra are deleted, modified, substituted, and/or added, and may be prepared by a method known to those of ordinary skill in the art. The II,-1Ra variant may comprise at least 5 or more, at least 10 or more, at least 15 or more, at least 20 or more, at least 30 or more, at least 40 or more, at least 50 or more, at least 60 or more, at least 70 or more, at least 80 or more, at least 90 or more, or 100 or more amino acids of the wild type IL-1Ra.

The term "IL-1Ra" or "IL-1 receptor antagonist (II,-1Ra)", unless otherwise stated, refers to any wild type II,-1Ra obtained from any vertebrate source, including mammals, for example, primates (e.g., humans) and rodents (e.g., mice and rats). II,-1Ra may be obtained from animal cells, and also includes one obtained from recombinant cells capable of producing IL-1Ra. In addition, II,-1Ra may be a wild type II,-1Ra or a fragment thereof.

### Second fusion protein comprising IL-1 receptor antagonist and Fc region and dimer thereof

In the present invention, the IL-1 receptor antagonist or a fragment thereof may be a dimer of a second fusion protein further comprising an Fc region fragment or a variant thereof.

Specifically, the second fusion protein comprising an IL-1 receptor antagonist or a fragment thereof, and an Fc region fragment or a variant thereof may consist of the following structural formula (V) or (VI):
N'-[linker (6)]o-Fc region fragment or variant thereof-[linker (7)]p-B-C' (V)
N'-B-[linker (8)]q-Fc region fragment or variant thereof-C' (VI)
wherein, in the structural formulas (V) and (VI),
N' is the N terminus of the fusion protein,
C' is the C terminus of the fusion protein,
B is an IL-1 receptor antagonist or a fragment thereof,
the linker (6), linker (7), and linker (8) are peptide linkers, and
o, p, and q are each independently 0 or 1.

At that time, the IL-1 receptor antagonist or a fragment thereof, and the Fc region fragment or a variant thereof are the same as described above.

The peptide linker (6) may consist of 5 to 80 consecutive amino acids, 5 to 50 consecutive amino acids, 5 to 40 consecutive amino acids, 5 to 30 consecutive amino acids, or 5 to 20 amino acids. In one embodiment, the peptide linker (6) may consist of 12 amino acids. In addition, the peptide linker (6) may comprise at least one cysteine. Specifically, it may comprise one, two, or three cysteines. In addition, the peptide linker (6) may be derived from the hinge of an immunoglobulin. In one embodiment, the peptide linker (6) may be a peptide linker consisting of the amino acid sequence of SEQ ID NO: 31.

The peptide linker (7) may consist of 1 to 50 consecutive amino acids, or 3 to 30 consecutive amino acids, or 5 to 20 amino acids. The peptide linker (7) may comprise (G4S)n (where n is an integer of 1 to 10), and may further comprise (G)n. At that time, in (G4S)n and (G)n, n may be each independently 1, 2, 3, 4, 5, 6, 7, 8, 9, or 10. In addition, the linker (7) may be a GS3 linker. In one embodiment, the peptide linker (7) may be a peptide linker consisting of the amino acid sequence of SEQ ID NO: 7. In one embodiment, the peptide linker (7) may be a peptide linker consisting of the amino acid sequence of SEQ ID NO: 26.

The peptide linker (8) may consist of 5 to 80 consecutive amino acids, 10 to 70 consecutive amino acids, 15 to 60 consecutive amino acids, 20 to 50 consecutive amino acids, or 25 to 40 amino acids. In one embodiment, the peptide linker (8) may consist of 30 amino acids. In addition, the peptide linker (8) may comprise at least one cysteine. Specifically, it may comprise one, two, or three cysteines. In addition, the peptide linker (8) may be derived from the hinge of an immunoglobulin. For example, the hinge may be selected from the hinge regions of various IgG subtype antibodies. In addition, the hinge may be a form in which some amino acids in the hinge region derived from an immunoglobulin are substituted with other amino acids, or may be a sequence in which some amino acid sequences are added. In one embodiment, the peptide linker (8) may be a peptide linker consisting of the amino acid sequence of SEQ ID NO: 5 or SEQ ID NO: 31.

Each amino acid sequence of the second fusion protein is shown in Table 2 below. In addition, one specific example of the second fusion protein (IgG4 Fc-IL-1Ra, GI210B1C1) may have the amino acid sequence of SEQ ID NO: 24.

**[Table 2]**

| Item | Amino acid sequence | SEQ ID NO |
|---|---|---|
| fourth linker | ESKYGPPCPPCP | 31 |
| hIgG4 Fc | APEAAGGPSVFLFPPKPKDQLMISRTPEVTCVVVDVSQEDPEVQFNWYVDGVEVHNAKTKP REEQFNSTYRVVSVLTVLHQDWLNGKEYKCKVSNKGLPSSIEKTISKAKGQPREPQVYTLP PSQEEMTKNQVSLTCLVKGFYPSDIAVEWESNGQPENNYKTTPPVLDSDGSFFLYSRLTVD KSRWQEGNVFSCSVLHEALHNHYTQKSLSLSLG | 6 |
| Second linker | GGGGSGGGGSGGGGSGG | 7 |
| IL-1Ra | RPSGRKSSKMQAFRIWDVNQKTFYLRNNQLVAGYLQGPNVNLEEKIDVVPIEPHALFLGIH GGKMCLSCVKSGDETRLQLEAVNITDLSENRKQDKRFAFIRSDSGPTTSFESAACPGWFLC TAMEADQPVSLTNMPDEGVMVTKFYFQEDE | 8 |
| GI210B1C1 (IgG4 Fc-IL-1Ra) | ESKYGPPCPPCPAPEAAGGPSVFLFPPKPKDQLMISRTPEVTCVVVDVSQEDPEVQFNWYV DGVEVHNAKTKPREEQFNSTYRVVSVLTVLHQDWLNGKEYKCKVSNKGLPSSIEKTISKAK GQPREPQVYTLPPSQEEMTKNQVSLTCLVKGFYPSDIAVEWESNGQPENNYKTTPPVLDSD GSFFLYSRLTVDKSRWQEGNVFSCSVLHEALHNHYTQKSLSLSLGGGGGSGGGGSGGGGSG GRPSGRKSSKMQAFRIWDVNQKTFYLRNNQLVAGYLQGPNVNLEEKIDVVPIEPHALFLGI HGGKMCLSCVKSGDETRLQLEAVNITDLSENRKQDKRFAFIRSDSGPTTSFESAACPGWFL CTAMEADQPVSLTNMPDEGVMVTKFYFQEDE | 24 |

In the present invention, the second fusion protein comprising an IL-1 receptor antagonist or a fragment thereof, and an Fc region fragment or a variant thereof may exist as a dimer in which the two second fusion proteins are linked. At that time, the second fusion protein may be linked by cysteine included in the linker located at the N terminus of the Fc region fragment or a variant thereof. At that time, the linker may comprise the hinge region of an immunoglobulin. The Fc region fragment may be an Fc region fragment derived from IgG4 or a variant thereof.

The second fusion protein may be linked by cysteine included in the linker located at the N terminus. At that time, the linker may comprise the hinge region of an immunoglobulin.

### Third fusion protein comprising GLP-1 and IL-1 receptor antagonist and dimer thereof

In addition, in the present invention, the GLP-1 or a variant thereof, and the IL-1 receptor antagonist or a fragment thereof may be a third fusion protein linked by a linker.

The linker connects two proteins. One specific example of the linker may include 1 to 50 amino acids, albumin or a fragment thereof, or an Fc region of an immunoglobulin, etc. Specifically, in the present invention, the linker may include an Fc region fragment or a variant thereof. The Fc region fragment or a variant thereof is the same as described above. In the present invention, one specific example of the Fc region fragment or a variant thereof may comprise the amino acid sequence of SEQ ID NO: 6.

The third fusion protein may be a form in which GLP-1 or a variant thereof and an IL-1 receptor antagonist or a fragment thereof are linked through an Fc region fragment or a variant thereof. At that time, the Fc region fragment or a variant thereof, GLP-1 or a variant thereof, and the IL-1 receptor antagonist or a fragment thereof may be linked through a peptide linker.

Specifically, the third fusion protein may consist of the following structural formula (I) or (II):
N'-X-[linker (1)]o-Fc region fragment or variant thereof-[linker (2)]p-Y-C' (I)
N'-Y-[linker (1)]o-Fc region fragment or variant thereof-[linker (2)]p-X-C' (II)
wherein, in the structural formulas (I) and (II),
N' is the N terminus of the fusion protein,
C' is the C terminus of the fusion protein,
X is GLP-1 or a variant thereof,
Y is an IL-1 receptor antagonist or a fragment thereof,
the linker (1) and linker (2) are peptide linkers, and
o and p are each independently 0 or 1.

At that time, the IL-1 receptor antagonist or a fragment thereof, and the Fc region fragment or a variant thereof are the same as described above.

The peptide linker (1) may consist of 5 to 80 consecutive amino acids, 10 to 70 consecutive amino acids, 15 to 60 consecutive amino acids, 20 to 50 consecutive amino acids, or 25 to 40 amino acids. In one embodiment, the peptide linker (1) may consist of 30 amino acids. In addition, the peptide linker (1) may comprise at least one cysteine. Specifically, it may comprise one, two, or three cysteines. In addition, the peptide linker (1) may be derived from the hinge of an immunoglobulin. In one embodiment, the peptide linker (1) may be a peptide linker consisting of the amino acid sequence of SEQ ID NO: 5 or SEQ ID NO: 31.

The peptide linker (2) may consist of 1 to 50 consecutive amino acids, or 3 to 30 consecutive amino acids, or 5 to 20 amino acids. The peptide linker (2) may comprise (G4S)n (where n is an integer of 1 to 10), and may further comprise (G)n. At that time, in (G4S)n and (G)n, n may be each independently 1, 2, 3, 4, 5, 6, 7, 8, 9, or 10. In one embodiment, the peptide linker (2) may be a peptide linker consisting of the amino acid sequence of SEQ ID NO: 7 or SEQ ID NO: 26.

Each amino acid sequence of the third fusion protein is shown in Table 3 below. In addition, one specific example of the third fusion protein (GLP-1-IgG4 Fc-IL-1Ra, GI210B1) may have the amino acid sequence of SEQ ID NO: 10.

**[Table 3]**

| Item | Amino acid sequence | SEQ ID NO |
|---|---|---|
| hGLP-1 | HAEGTFTSDVSSYLEGQAAKEFIAWLVKGR | 1 |
| GLP-1 variant-1 | HGEGTFTSDVSSYLEEQAAKEFIAWLVKGGG | 2 |
| GLP-1 variant-2 | HGEGTFTSDLSKQMEEEAVRLFIEWLKNGGPSSGAPPPS | 3 |
| GLP-1 variant-3 | HAEGTFTSDVSSYLEGQAAKEFIAWLVRGRG | 4 |
| first linker | GGGGSGGGGSGGGGSGGAESKYGPPCPPCP | 5 |
| hIgG4 Fc | APEAAGGPSVFLFPPKPKDQLMISRTPEVTCVVVDVSQEDPEVQFNWYVDGVEVH NAKTKPREEQFNSTYRVVSVLTVLHQDWLNGKEYKCKVSNKGLPSSIEKTISKAK GQPREPQVYTLPPSQEEMTKNQVSLTCLVKGFYPSDIAVEWESNGQPENNYKTTP PVLDSDGSFFLYSRLTVDKSRWQEGNVFSCSVLHEALHNHYTQKSLSLSLG | 6 |
| Second linker | GGGGSGGGGSGGGGSGG | 7 |
| IL-1Ra | RPSGRKSSKMQAFRIWDVNQKTFYLRNNQLVAGYLQGPNVNLEEKIDVVPIEPHA LFLGIHGGKMCLSCVKSGDETRLQLEAVNITDLSENRKQDKRFAFIRSDSGPTTS FESAACPGWFLCTAMEADQPVSLTNMPDEGVMVTKFYFQEDE | 8 |
| GI210B1 (GLP-1-IgG4 Fc-IL-1Ra) | HGEGTFTSDVSSYLEEQAAKEFIAWLVKGGGGGGGSGGGGSGGGGSGGAESKYGP PCPPCPAPEAAGGPSVFLFPPKPKDQLMISRTPEVTCVVVDVSQEDPEVQFNWYV DGVEVHNAKTKPREEQFNSTYRVVSVLTVLHQDWLNGKEYKCKVSNKGLPSSIEK TISKAKGQPREPQVYTLPPSQEEMTKNQVSLTCLVKGFYPSDIAVEWESNGQPEN NYKTTPPVLDSDGSFFLYSRLTVDKSRWQEGNVFSCSVLHEALHNHYTQKSLSLS LGGGGGSGGGGSGGGGSGGRPSGRKSSKMQAFRIWDVNQKTFYLRNNQLVAGYLQ GPNVNLEEKIDVVPIEPHALFLGIHGGKMCLSCVKSGDETRLQLEAVNITDLSEN RKQDKRFAFIRSDSGPTTSFESAACPGWFLCTAMEADQPVSLTNMPDEGVMVTKF YFQEDE | 10 |

In the present invention, the third fusion protein consisting of GLP-1 or a variant thereof, and an IL-1 receptor antagonist or a fragment thereof may exist as a dimer in which the two third fusion proteins are linked. At that time, the third fusion protein may be linked by cysteine included in the linker connecting GLP-1 or a variant thereof and an immunoglobulin Fc region fragment or a variant thereof. The third fusion protein may be linked by cysteine included in the linker connecting an IL-1 receptor antagonist or a fragment thereof and an Fc region fragment or a variant thereof. At that time, the linker may comprise the hinge region of an immunoglobulin.

The composition comprising GLP-1 or a variant thereof, and an IL-1 receptor antagonist or a fragment thereof as active ingredients, according to the present invention, may be used as a pharmaceutical composition for preventing or treating degenerative brain diseases.

As used herein, the term "degenerative brain disease" refers to a disease that occurs in the brain among degenerative diseases that occur with age. Currently, the cause of the disease has not been clearly identified, but it is known to be caused by the death of brain nerve cells and/or problems with the formation or function of synapses that transmit information between brain nerve cells.

The degenerative brain disease may be any one selected from the group consisting of Alzheimer's disease, Parkinson's disease, Huntington's disease, mild cognitive impairment, cerebral amyloid angiopathy, Down syndrome, amyloid stroke, systemic amyloid disease, Dutch amyloidosis, Niemann-Pick disease, senile dementia, amyotrophic lateral sclerosis, spinocerebellar atrophy, Tourette's syndrome, Friedreich's ataxia, Machado-Joseph's disease, lewy body dementia, dystonia, progressive supranuclear palsy, and frontotemporal dementia, but is not limited thereto.

A preferred dosage of the pharmaceutical composition varies depending on the patient's condition and body weight, severity of disease, form of drug, route and duration of administration and may be appropriately selected by those of ordinary skill in the art. In the pharmaceutical composition for preventing or treating degenerative brain diseases of the present invention, the GLP-1 or a variant thereof, and the IL-1 receptor antagonist or a fragment thereof may be contained in any amount (effective amount) depending on application, dosage form, blending purpose, and the like, as long as they can exhibit a therapeutic activity for degenerative brain diseases or, in particular, can exhibit a therapeutic effect on Alzheimer's disease. A conventional effective amount thereof may be determined within a range of 0.001% to 20.0% by weight, based on the total weight of the composition. At that time, the term "effective amount" refers to an amount of the above dimer protein that can induce an effect of improving or treating the condition of a disease, especially an effect of improving or treating the condition of Alzheimer's disease. Such an effective amount can be experimentally determined within the scope of common knowledge of those of ordinary skill in the art.

As used herein, the term "treatment" may be used to mean both therapeutic and prophylactic treatment. At that time, prophylaxis may be used to mean that a pathological condition or disease of a subject is alleviated or mitigated. In one embodiment, the term "treatment" includes both application or any form of administration for treating a disease in a mammal, including a human. In addition, the term includes inhibiting or slowing down the progression of a disease; and includes meanings of restoring or repairing impaired or lost function so that a disease is partially or completely alleviated; stimulating inefficient processes; or alleviating a serious disease.

Pharmacokinetic parameters such as bioavailability and underlying parameters such as clearance rate may also affect efficacy. Therefore, "improved efficacy" (for example, improvement in efficacy) may be due to improved pharmacokinetic parameters.

Meanwhile, the pharmaceutical composition of the present invention is administered in a therapeutically effective amount.

As used herein, the term "administration" means introducing a predetermined substance into a subject by an appropriate method, and the composition may be administered through any general route as long as it can reach the target tissue. The composition may be administered by intraperitoneal administration, intravenous administration, intramuscular administration, subcutaneous administration, intradermal administration, topical administration, intranasal administration, intrapulmonary administration, or intrarectal administration, but is not limited thereto.

The active ingredients of the pharmaceutical composition, GLP-1 or a variant thereof, and an IL-1 receptor antagonist or a fragment thereof may be simultaneously administered in combination in one formulation. The GLP-1 or a variant thereof, and the IL-1 receptor antagonist or a fragment thereof may be administered simultaneously in separate formulations, or may be administered in combination sequentially or in reverse order. In addition, the GLP-1 or a variant thereof, and the IL-1 receptor antagonist or a fragment thereof may be administered in the form of a dimer of a fusion protein linked through a linker. At that time, the dimer of a fusion protein in which GLP-1 or a variant thereof, and an IL-1 receptor antagonist or a fragment thereof are linked through a linker is the same as described above.

As used herein, the term "therapeutically effective amount" or "pharmaceutically effective amount" refers to an amount of a compound or composition effective to prevent or treat the disease in question, which is sufficient to treat the disease at a reasonable benefit/risk ratio applicable to medical treatment and does not cause adverse effects. A level of the effective amount may be determined depending on factors including the patient's health condition, type and severity of disease, activity of drug, the patient's sensitivity to drug, mode of administration, time of administration, route of administration and excretion rate, duration of treatment, combined or simultaneously used drugs, and other factors well known in the medical field. In one embodiment, the therapeutically effective amount means an amount of drug effective to treat degenerative brain diseases.

A preferred dosage of the pharmaceutical composition may be appropriately selected and administered depending on the patient's condition, body weight, sex, age, severity of the patient, and route of administration. For example, when the active ingredients of the pharmaceutical composition, GLP-1 or a variant thereof, and an IL-1 receptor antagonist or a fragment thereof are simultaneously administered in combination in one formulation, the dosage of the pharmaceutical composition may be in the range of 0.01 µg/kg to 10 g/kg per day, or may be in the range of 0.01 mg/kg to 1 g/kg per day. When the GLP-1 or a variant thereof, and the IL-1 receptor antagonist or a fragment thereof are administered in separate formulations, the GLP-1 or a variant thereof, and the IL-1 receptor antagonist or a fragment thereof may be administered in the range of 0.01 µg/kg to 10 g/kg per day, or in the range of 0.01 mg/kg to 1 g/kg, respectively. In addition, when the GLP-1 or a variant thereof, and the IL-1 receptor antagonist or a fragment thereof are administered in the form of a fusion protein dimer, the fusion protein dimer may be administered in the range of 0.01 µg/kg to 10 g/kg per day, or in the range of 0.01 mg/kg to 1 g/kg. Such a dosage should not be construed as limiting the scope of the present invention in any aspect.

A subject to which the pharmaceutical composition can be applied (prescribed) is a mammal and a human, and in particular, is preferably a human. In addition to the active ingredient, the pharmaceutical composition of the present invention may further comprise any compound or natural extract, which is known to have a therapeutic effect on degenerative brain diseases. At that time, the compound or natural extract may be administered simultaneously or sequentially with the pharmaceutical composition.

The pharmaceutical composition may further comprise a pharmaceutically acceptable carrier. At that time, the term "pharmaceutically acceptable" means that the carrier does not have more toxicity than the subject to be applied (prescribed) can adapt while not inhibiting activity of the active ingredient. For example, distilled water, alcohol, fat, wax, and inert solids may be included as carriers, but are not limited thereto. A pharmacologically acceptable adjuvant (buffering agent, dispersing agent) may also be included in the pharmaceutical composition.

Specifically, by including a pharmaceutically acceptable carrier in addition to an active ingredient, the pharmaceutical composition may be prepared into a parenteral formulation depending on its route of administration using conventional a method known in the art.

When the pharmaceutical composition is prepared into a parenteral formulation, it may be made into preparations in the form of injections, transdermal patches, nasal inhalants, or suppositories with suitable carriers according to a method known in the art. In a case of being made into injections, sterile water, ethanol, polyol such as glycerol or propylene glycol, or a mixture thereof may be used as a suitable carrier; and an isotonic solution, such as Ringer's solution, phosphate buffered saline (PBS) containing triethanol amine or sterile water for injection, and 5% dextrose, or the like may preferably be used. Formulation of pharmaceutical compositions is known in the art, and reference may specifically be made to Remington's Pharmaceutical Sciences (19th ed., 1995) and the like. This document is considered part of the present specification.

In another aspect of the present invention, there is provided a use of a pharmaceutical composition comprising GLP-1 or a variant thereof, and an IL-1 receptor antagonist or a fragment thereof for the prevention or treatment of degenerative brain diseases. Here, the GLP-1 or a variant thereof, the IL-1 receptor antagonist or a fragment thereof, the pharmaceutical composition, the prevention, and the treatment are the same as described above.

In another aspect of the present invention, there is provided a use of a pharmaceutical composition comprising GLP-1 or a variant thereof, and an IL-1 receptor antagonist or a fragment thereof for manufacture of a medicament for preventing or treating degenerative brain diseases. Here, the GLP-1 or a variant thereof, the IL-1 receptor antagonist or a fragment thereof, the pharmaceutical composition, the prevention, and the treatment are the same as described above.

In another aspect of the present invention, there is provided a method for preventing or treating degenerative brain diseases, comprising administering a pharmaceutical composition comprising GLP-1 or a variant thereof, and an IL-1 receptor antagonist or a fragment thereof to a subject. Here, the GLP-1 or a variant thereof, the IL-1 receptor antagonist or a fragment thereof, the pharmaceutical composition, the degenerative brain disease, the prevention, and the treatment are the same as described above.

The "subject" may be a mammal, and preferably, may be a human. In addition, the subject may be a patient suffering from a degenerative brain disease or may be a subject with a high probability of suffering from a degenerative brain disease.

Route of administration, dosage, and frequency of administration of the composition may vary depending on the patient's condition and the presence or absence of side effects, and thus the composition may be administered to a subject in various ways and amounts. The optimal administration method, dosage, and frequency of administration can be selected in an appropriate range by those of ordinary skill in the art. In addition, the composition may be administered in combination with any compound or natural extract, which is known to have a therapeutic effect on degenerative brain diseases, or may be formulated in the form of combination preparations with other drugs.

### Polynucleotide encoding fusion protein

In another aspect of the present invention, there is provided a polynucleotide encoding the first fusion protein, the second fusion protein, or the third fusion protein. At that time, the first fusion protein, the second fusion protein, and the third fusion protein are the same as described above. In one embodiment, a polynucleotide encoding the first fusion protein according to the present invention may comprise the nucleotide sequence of SEQ ID NO: 23. In one embodiment, a polynucleotide encoding the second fusion protein according to the present invention may comprise the nucleotide sequence of SEQ ID NO: 25. In one embodiment, a polynucleotide encoding the third fusion protein according to the present invention may comprise the nucleotide sequence of SEQ ID NO: 20.

In addition, if the polynucleotide encodes the same polypeptide, one or more bases may be mutated by substitution, deletion, insertion, or a combination thereof. When the polynucleotide sequence is prepared by chemical synthesis, a synthesis method well known in the art, for example, the method described in the literature (Engels and Uhlmann, Angew Chem IntEd Engl., 37:73-127, 1988), may be used, and may include triester, phosphite, phosphoramidite and H-phosphate methods, PCR, and other autoprimer methods, oligonucleotide synthesis methods on a solid support, and the like.

According to one embodiment, the polynucleotide may include a nucleotide sequence having at least about 70%, at least about 75%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, or at least about 100% identity to the nucleotide sequence of SEQ ID NO: 23.

According to one embodiment, the polynucleotide may include a nucleotide sequence having at least about 70%, at least about 75%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, or at least about 100% identity to the nucleotide sequence of SEQ ID NO: 25.

According to one embodiment, the polynucleotide may include a nucleotide sequence having at least about 70%, at least about 75%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, or at least about 100% identity to the nucleotide sequence of SEQ ID NO: 20.

The polynucleotide may further comprise a signal sequence or a leader sequence. As used herein, the term "signal sequence" refers to a nucleic acid encoding a signal peptide that directs secretion of a target protein. The signal peptide is translated and then cleaved in a host cell. Specifically, the signal sequence of the present invention is a polynucleotide encoding an amino acid sequence that initiates migration of a protein across the endoplasmic reticulum (ER) membrane.

Signal sequences are well known in the art for their characteristics. Such signal sequences typically contain 16 to 30 amino acid residues, and may contain more or fewer amino acid residues than such amino acid residues. A typical signal peptide is composed of three regions, that is, a basic N-terminal region, a central hydrophobic region, and a more polar C-terminal region. The central hydrophobic region contains 4 to 12 hydrophobic residues that cause the signal sequence to be immobilized during migration of an immature polypeptide through the membrane lipid bilayer.

After initiation, signal sequences are cleaved in the lumen of ER by cellular enzymes, commonly known as signal peptidases. At that time, the signal sequence may be a secretory signal sequence of tPa (tissue plasminogen activator), HSV gDs (signal sequence of Herpes simplex virus glycoprotein D), IgG signal sequence, or a growth hormone. Preferably, a secretory signal sequence used in higher eukaryotic cells including mammals and the like may be used. Signal sequences useful in the present invention include antibody light chain signal sequences, such as antibody 14.18 (Gillies et al., J. Immunol. Meth 1989. 125:191-202), antibody heavy chain signal sequences, such as MOPC141 antibody heavy chain signal sequence (Sakano et al., Nature, 1980. 286: 676-683), and other signal sequences known in the art (see, for example, Watson et al., Nucleic Acid Research, 1984. 12:5145-5164). In one embodiment, the signal sequence may comprise the amino acid sequence of SEQ ID NO: 9.

### Vector loaded with polynucleotide

In another aspect of the present invention, there is provided a vector loaded with a polynucleotide encoding the first fusion protein, the second fusion protein, or the third fusion protein, respectively. The first fusion protein, the second fusion protein, and the third fusion protein are the same as described above. In one embodiment, the vector may comprise the nucleotide sequence of SEQ ID NO: 23, SEQ ID NO: 25, or SEQ ID NO: 20.

As used herein, the term "vector" may be introduced into a host cell to be recombined with and inserted into the genome of the host cell. Alternatively, the vector is understood as nucleic acid means containing a nucleotide sequence which is autonomously replicable as an episome. The vectors include linear nucleic acids, plasmids, phagemids, cosmids, RNA vectors, viral vectors, mini-chromosomes, and analogs thereof. Examples of the viral vector include, but are not limited to, retroviruses, adenoviruses, and adeno-associated viruses.

Specifically, the vector may be plasmid DNA, phage DNA, etc., commercially developed plasmids (pUC18, pBAD, pIDTSAMRT-AMP, etc.), E. coli derived plasmids (pYG601BR322, pBR325, pUC118, pUC119, etc.), Bacillus subtilis derived plasmids (pUB110, pTP5, etc.), yeast derived plasmids (YEp13, YEp24, YCp50, etc.), phage DNA (Charon4A, Charon21A, EMBL3, EMBL4, λgt10, λgt11, λZAP, etc.), animal viral vectors (retrovirus, adenovirus, vaccinia virus, etc.), insect viral vectors (baculovirus, etc.), and the like. Since the vector exhibits different protein expression levels and the like depending on the host cell, it is preferable to select and use the most suitable host cell for the purpose.

In addition, the plasmid may contain a selectable marker such as an antibiotic resistance gene, and host cells maintaining the plasmid may be cultured under selective conditions.

As used herein, the term "gene expression" or "expression" of a target protein is understood to mean transcription of DNA sequences, translation of mRNA transcripts, and secretion of fusion protein products or fragments thereof. A useful expression vector may be RcCMV (Invitrogen, Carlsbad) or a variant thereof. The expression vectors may contain a human cytomegalovirus (CMV) promoter for promoting continuous transcription of a target gene in mammalian cells, and a fetal bovine growth hormone polyadenylation signal sequence for increasing the stability level of RNA after transcription.

### Transformed cell

In another aspect of the present invention, there is provided a transformed cell into which an expression vector loaded with a polynucleotide encoding the first fusion protein, the second fusion protein, or the third fusion protein is introduced, respectively. The first fusion protein, the second fusion protein, and the third fusion protein are the same as described above.

As used herein, the term "transformed cell" refers to prokaryotic cells and eukaryotic cells into which a recombinant expression vector can be introduced. The transformed cell may be prepared by introducing a vector into a host cell and transforming it. In addition, the fusion protein according to the present invention may be produced by expressing the polynucleotide contained in the vector.

The transformation may be performed by various methods. As long as it can produce the fusion protein of the present invention, it is not particularly limited thereto. Specifically, as the transformation method, CaCl₂ precipitation method, Hanahan method whose efficiency has been increased by using a reducing agent such as dimethyl sulfoxide (DMSO) in CaCl₂ precipitation method, electroporation, calcium phosphate precipitation method, protoplast fusion method, agitation method using silicon carbide fiber, agrobacteria mediated transformation method, transformation method using PEG, dextran sulfate, lipofectamine and dry/inhibition mediated transformation method, or the like may be used. In addition, by using the infection as a means, a target object can be delivered into a cell using virus particles. In addition, the vector can be introduced into the host cell by gene bombardment or the like.

In addition, as long as the host cell used for the production of the transformed cell can also produce the fusion protein of the present invention, it is not particularly limited thereto. Specifically, the host cells may include, but are not limited to, prokaryotic cells, eukaryotic cells, and cells of mammalian, plant, insect, fungal, or bacterial origin. As an example of the prokaryotic cells, E. coli may be used. In addition, as an example of the eukaryotic cells, yeast may be used. In addition, as the mammalian cells, CHO cells, F2N cells, COS cells, BHK cells, Bowes melanoma cells, HeLa cells, 911 cells, AT1080 cells, A549 cells, SP2/0 cells, human lymphoblastoid, NSO cells, HT-1080 cells, PERC.6 cells, HEK 293 cells, HEK293T cells, or the like may be used, but are not limited thereto, and any cells which are known to those of ordinary skill in the art to be usable as mammalian host cells may be used.

In addition, for optimization of properties of a fusion protein as a therapeutic agent or for any other purpose, glycosylation pattern of the antibody (for example, sialic acids, fucosylations, glycosylations) may be adjusted by manipulating, through a method known to those of ordinary skill in the art, glycosylation-associated genes possessed by host cells.

### Method of producing fusion protein

In another aspect of the present invention, there is provided a method of producing the fusion protein, comprising the steps of: i) culturing the transformed cell; and ii) obtaining the fusion protein from the culture solution.

As used herein, the term "culture" refers to a method of growing microorganisms in an appropriately artificially controlled environmental condition.

The method of culturing the transformed cells may be carried out using a method well known in the art. Specifically, the culture is not particularly limited as long as it can express and produce the antibody of the present invention. Specifically, the culture may be carried out in a batch process, or carried out continuously in a fed batch or repeated fed batch process.

In addition, the step of obtaining the fusion protein from the culture product may be performed by a method known in the art. Specifically, the obtaining method is not particularly limited as long as the produced fusion protein of the present invention can be obtained. Preferably, the obtaining method may be a method such as centrifugation, filtration, extraction, spraying, drying, evaporation, precipitation, crystallization, electrophoresis, fractional dissolution (for example, ammonium sulfate precipitation), chromatography (for example, ion exchange, affinity, hydrophobicity, and size exclusion).

### II. Bispecific antibody in which GLP-1 is linked to anti-IL-1 antibody

In another aspect of the present invention, there is provided a bispecific antibody in which GLP-1 or a variant thereof is linked to an anti-IL-1 antibody or a fragment thereof. The IL-1 and GLP-1 or a variant thereof are the same as described above.

As used herein, the term "antibody" refers to an immunoglobulin molecule that reacts immunologically with a specific antigen, and refers to a protein molecule that specifically recognizes the antigen. The heavy and light chains of an immunoglobulin may each include a constant region and a variable region. The light and heavy chain variable regions of an immunoglobulin include three variable regions called complementarity determining regions (CDRs) and four framework regions (FRs). The CDR is an antigen binding site that mainly binds to the epitope of the antigen. The CDRs of each chain are typically called CDR1, CDR2, and CDR3 sequentially, starting from the N terminus, and are identified by the chain on which the specific CDR is located.

The antibody includes a whole antibody, a monoclonal antibody, a polyclonal antibody, a single domain antibody, a single chain antibody, a multispecific antibody, a human antibody, a humanized antibody, a chimeric antibody, an intrabody, an scFv, a Fab fragment, a F(ab') fragment, a Fv linked by disulfide bond (sdFv), and an epitope-binding fragment of any of the above, but is not limited thereto.

As used herein, the term "antibody fragment" may include a Fab fragment, a Fab' fragment, a F(ab')2 fragment having antigen-binding activity, as well as an scFv fragment, which is an Fv fragment that binds to an antigen. The Fv fragment is the minimal antibody fragment that contains a heavy chain variable region and a light chain variable region, without a constant region, and retains all antigen-binding sites.

As used herein, the term "anti-IL-1 antibody" refers to an antibody capable of specifically binding to IL-1. The form of the antibody may include both whole antibodies and antibody fragments thereof. In the present specification, the "anti-IL-1 antibody" can be used interchangeably with the "antibody specific for IL-1." Specifically, the anti-IL-1 antibody may specifically bind to IL-1β.

As used herein, the term "bispecific antibody" refers to a substance that binds to at least one or more target or antigen. In the present invention, the bispecific antibody may comprise an antigen binding site that specifically binds to IL-1, and GLP-1 or a variant thereof. The bispecific antibody of the present invention may comprise an antibody that specifically binds to IL-1 or a fragment thereof, and GLP-1 or a variant thereof.

The heavy chain variable regions of the bispecific antibody, HCDR1, HCDR2, and HCDR3, may comprise the amino acid sequences of SEQ ID NO: 39, SEQ ID NO: 40, and SEQ ID NO: 41, respectively. At that time, the light chain variable regions of the bispecific antibody, LCDR1, LCDR2, and LCDR3, may comprise the amino acid sequences of SEQ ID NO: 42, SEQ ID NO: 43, and SEQ ID NO: 44, respectively.

In one embodiment of the present invention, the bispecific antibody may comprise a heavy chain variable region comprising the amino acid sequence of SEQ ID NO: 32 and a light chain variable region comprising the amino acid sequence of SEQ ID NO: 34.

The heavy chain variable region of the antibody may comprise or consist of an amino acid sequence having about 90% or more, about 91% or more, about 92% or more, about 93% or more, about 94% or more, about 95% or more, about 96% or more, about 97% or more, about 98% or more, about 99% or more, or 100% identity to the amino acid sequence of SEQ ID NO: 32. In addition, the light chain variable region of the antibody may comprise or consist of an amino acid sequence having about 90% or more, about 91% or more, about 92% or more, about 93% or more, about 94% or more, about 95% or more, about 96% or more, about 97% or more, about 98% or more, about 99% or more, or 100% identity to the amino acid sequence of SEQ ID NO: 34.

In addition, in the present invention, the anti-IL-1 antibody may further comprise an Fc region fragment or a variant thereof. The Fc region fragment or a variant thereof is the same as described above. In one embodiment of the present invention, the Fc region fragment or a variant thereof may comprise the amino acid sequence of SEQ ID NO: 6. At that time, the antigen binding site of the anti-IL-1 antibody and the Fc region fragment or a variant thereof may be linked through a linker. The linker may consist of 5 to 80 consecutive amino acids, 10 to 70 consecutive amino acids, 15 to 60 consecutive amino acids, 20 to 50 consecutive amino acids, or 25 to 40 amino acids. In one embodiment, the peptide linker may consist of 30 amino acids. In addition, the peptide linker may comprise at least one cysteine. Specifically, it may comprise one, two, or three cysteines. In addition, the peptide linker may be derived from the hinge of an immunoglobulin. In one embodiment, the peptide linker may be a peptide linker consisting of the amino acid sequence of SEQ ID NO: 5 or SEQ ID NO: 31.

In addition, GLP-1 or a variant thereof may be linked to a heavy chain or a light chain of an anti-IL-1 antibody. Specifically, GLP-1 or a variant thereof may be linked to the N terminus of the heavy chain or the C terminus of the Fc region. In addition, GLP-1 or a variant thereof may be linked to the N terminus or C terminus of the light chain. At that time, GLP-1 or a variant thereof may be linked through a peptide linker.

In addition, GLP-1 or a variant thereof may be a form in which 1, 2, 3, 4, 5, 6, 7, or 8 are linked to the anti-IL-1 antibody.

Specifically, the bispecific antibody in which GLP-1 or a variant thereof is linked to an anti-IL-1 antibody or a fragment thereof may consist of the following structural formulas (VII) and (VIII):
N'-[D]t-[linker (9)]u-E-[linker (10)]v-Fc region fragment or variant thereof-[linker (9)]w-[D]x-C' (VII)
N'-[D]y-[linker (9)]z-E'-[linker (9)]a-[D]x-C' (VIII)
wherein, in the structural formulas (VII) and (VIII),
N' is the N terminus of the bispecific antibody,
C' is the C terminus of the bispecific antibody,
D is GLP-1 or a variant thereof,
E is the antigen binding site of the heavy chain of the anti-IL-1 antibody and comprises a variable region (VH) and a CH1 region,
E' is the antigen binding site of the light chain of the anti-IL-1 antibody and comprises a variable region (VL) and a constant region (CL),
the linker (9) and linker (10) are peptide linkers, and
t, u, v, w, x, y, z, and a are each independently 0 or 1.

At that time, GLP-1 or a variant thereof, the antigen binding site, the variable region, the constant region, and the anti-IL-1 antibody, and the Fc region fragment or a variant thereof are the same as described above. In addition, in the present invention, GLP-1 or a variant thereof may be linked to the C terminus of the heavy chain constant region (CH1) of an anti-IL-1 antibody through a peptide linker.

The peptide linker (9) may consist of 1 to 50 consecutive amino acids, or 1 to 30 consecutive amino acids, or 1 to 20 amino acids. In one embodiment, the peptide linker (9) may be (GS3)n (where n is an integer of 1 to 10). At that time, in (GS3)n, n may be 1, 2, 3, 4, 5, 6, 7, 8, 9, or 10. In one embodiment, the peptide linker (9) may be a peptide linker consisting of the amino acid sequence of SEQ ID NO: 26. In one embodiment, the peptide linker (9) may comprise (G4S)n (where n is an integer of 1 to 10), and may further comprise (G)n. At that time, in (G4S)n and (G)n, n may be each independently 1, 2, 3, 4, 5, 6, 7, 8, 9, or 10. In one embodiment, the peptide linker (9) may be a peptide linker consisting of the amino acid sequence of SEQ ID NO: 26 or SEQ ID NO: 7.

The linker (10) may consist of 5 to 80 consecutive amino acids, 10 to 70 consecutive amino acids, 15 to 60 consecutive amino acids, 20 to 50 consecutive amino acids, or 25 to 40 amino acids. In one embodiment, the peptide linker (10) may consist of 30 amino acids. In addition, the peptide linker (10) may comprise at least one cysteine. Specifically, it may comprise one, two, or three cysteines. In addition, the peptide linker (10) may be derived from the hinge of an immunoglobulin. In one embodiment, the peptide linker (10) may be a peptide linker consisting of the amino acid sequence of SEQ ID NO: 5 or SEQ ID NO: 31.

In one embodiment of the present invention, each amino acid sequence of the bispecific antibody (GLP-1-IgG4 Fc-anti-IL-1 antibody, GI212B2) is shown in Table 4 below.

**[Table 4]**

| Item | | Amino acid sequence | SEQ ID NO |
|---|---|---|---|
| GLP-1-anti-IL-1 HC-hIgG Fc | signal peptide(mIgG) | MEWSWVFLFFLSVTTGVHS | 9 |
| | hGLP-1 | HGEGTFTSDVSSYLEEQAAKEFIAWLVKGGG | 2 |
| | Third linker | GGGGSGGGGSGGGGS | 37 |
| | anti-IL-1 VH | | 32 |
| | anti-IL-1 CH1 | ASTKGPSVFPLAPCSRSTSESTAALGCLVKDYFPEPVTVS WNSGALTSGVHTFPAVLQSSGLYSLSSVVTVPSSSLGTKT YTCNVDHKPSNTKVDKRV | 33 |
| | First linker | ESKYGPPCPPCP | 31 |
| | hIgG4(F02K) | APEAAGGPSVFLFPPKPKDQLMISRTPEVTCVVVDVSQED PEVQFNWYVDGVEVHNAKTKPREEQFNSTYRVVSVLTVLH QDWLNGKEYKCKVSNKGLPSSIEKTISKAKGQPREPQVYT LPPSQEEMTKNQVSLTCLVKGFYPSDIAVEWESNGQPENN YKTTPPVLDSDGSFFLYSRLTVDKSRWQEGNVFSCSVLHE ALHNHYTQKSLSLSLGK | 38 |
| anti-hIL-1 LC(kappa) | signal peptide(mIgG) | MEWSWVFLFFLSVTTGVHS | 9 |
| | anti-IL-1 VL | | 34 |
| | anti-IL-1 kappa CL | | 35 |

In the present invention, the bispecific antibody may be in the form of a dimer in which the two fusion protein monomers, in which GLP-1 or a variant thereof is linked to a heavy chain of an anti-IL-1 antibody or a fragment thereof, are linked. In addition, the heavy chain of an anti-IL-1 antibody or a fragment thereof is linked to a light chain of an anti-IL-1 antibody or a fragment thereof, and thus is capable of specifically binding to IL-1. In addition, the fusion protein monomer may be linked by cysteine included in the linker. At that time, the linker may comprise the hinge region of an immunoglobulin.

### Polynucleotide encoding bispecific antibody

In another aspect of the present invention, there is provided a polynucleotide encoding a bispecific antibody in which GLP-1 or a variant thereof is linked to an anti-IL-1 antibody or a fragment thereof. The anti-IL-1 antibody or a fragment thereof, the GLP-1 or a variant thereof, and the bispecific antibody are the same as described above.

The polynucleotide may comprise the nucleotide sequence of SEQ ID NO: 28 that encodes GLP-1 and a heavy chain of an anti-IL-1 antibody region. In addition, the polynucleotide may comprise the nucleotide sequence of SEQ ID NO: 30 that encodes a light chain region of an anti-IL-1 antibody.

In addition, if the polynucleotide encodes the same polypeptide, one or more bases may be mutated by substitution, deletion, insertion, or a combination thereof. According to one embodiment, the polynucleotide may include a nucleotide sequence having at least about 70%, at least about 75%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, or at least about 100% identity to the nucleotide sequence of SEQ ID NO: 28.

According to one embodiment, the polynucleotide may include a nucleotide sequence having at least about 70%, at least about 75%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, or at least about 100% identity to the nucleotide sequence of SEQ ID NO: 30.

The method for chemically synthesizing the polynucleotide sequence is the same as described above.

The polynucleotide may further comprise a signal sequence or a leader sequence. The signal sequence is the same as described above. In one embodiment, the signal sequence may comprise the amino acid sequence of SEQ ID NO: 9.

### Vector loaded with polynucleotide

In another aspect of the present invention, there is provided a vector loaded with a polynucleotide encoding the bispecific antibody in which GLP-1 or a variant thereof is linked to an anti-IL-1 antibody or a fragment thereof. The GLP-1 or a variant thereof, the anti-IL-1 antibody or a fragment thereof, and the bispecific antibody are the same as described above.

In one embodiment, the polynucleotide may comprise the nucleotide sequence of SEQ ID NO: 28 that encodes GLP-1 and a heavy chain of an anti-IL-1 antibody region, and the nucleotide sequence of SEQ ID NO: 30 that encodes a light chain region of an anti-IL-1 antibody.

### Transformed cell

In another aspect of the present invention, there is provided a transformed cell into which an expression vector loaded with a polynucleotide encoding the bispecific antibody in which GLP-1 or a variant thereof is linked to an anti-IL-1 antibody or a fragment thereof is introduced. The bispecific antibody of the present invention may be produced by expressing the polynucleotide included in the vector. The GLP-1 or a variant thereof, the anti-IL-1 antibody or a fragment thereof, and the bispecific antibody, and the transformed cell are the same as described above.

The host cell used to produce the transformed cell is also not particularly limited as long as it is capable of producing the antibody of the present invention.

### Method of producing bispecific antibody

In another aspect of the present invention, there is provided a method of producing a bispecific antibody in which GLP-1 or a variant thereof is linked to an anti-IL-1 antibody or a fragment thereof, comprising the steps of: i) culturing the transformed cell; and ii) obtaining the bispecific antibody in which GLP-1 or a variant thereof is linked to an anti-IL-1 antibody or a fragment thereof from the culture solution. The transformed cell, the anti-IL-1 antibody or a fragment thereof, the GLP-1 or a variant thereof, and the bispecific antibody are the same as described above.

### Pharmaceutical composition comprising bispecific antibody

In another aspect of the present invention, there is provided a pharmaceutical composition for preventing or treating degenerative brain diseases, comprising the bispecific antibody in which GLP-1 or a variant thereof is linked to an anti-IL-1 antibody or a fragment thereof as an active ingredient. The bispecific antibody, the degenerative brain disease, the prevention, and the treatment are the same as described above.

A preferred dosage of the pharmaceutical composition may be in the range of 0.01 µg/kg to 10 g/kg per day, or in the range of 0.01 mg/kg to 1 g/kg, depending on the patient's condition, body weight, sex, age, severity of the patient, and route of administration. Such a dosage should not be construed as limiting the scope of the present invention in any aspect.

In another aspect of the present invention, there is provided a use of a bispecific antibody in which GLP-1 or a variant thereof is linked to an anti-IL-1 antibody or a fragment thereof and the pharmaceutical composition comprising the same as an active ingredient for the prevention or treatment of degenerative brain diseases. Here, the bispecific antibody, the pharmaceutical composition, the prevention, and the treatment are the same as described above.

In another aspect of the present invention, there is provided a use of a bispecific antibody in which GLP-1 or a variant thereof is linked to an anti-IL-1 antibody or a fragment thereof and the pharmaceutical composition comprising the same as an active ingredient for manufacture of a medicament for preventing or treating degenerative brain diseases. Here, the bispecific antibody, the pharmaceutical composition, the prevention, and the treatment are the same as described above.

In another aspect of the present invention, there is provided a method for preventing or treating degenerative brain diseases, comprising administering the bispecific antibody and the pharmaceutical composition comprising the same as an active ingredient to a subject. Here, the bispecific antibody, the pharmaceutical composition, the degenerative brain disease, the subject, the administration, the prevention, and the treatment are the same as described above.

### III. Fusion protein comprising IL-1 receptor antagonist or fragment thereof; and Fc region fragment or variant thereof, and dimer thereof

In another aspect of the present invention, there is provided a fusion protein comprising an IL-1 receptor antagonist or a fragment thereof; and an Fc region fragment or a variant thereof. At that time, the IL-1 receptor antagonist or a fragment thereof, and the Fc region fragment or a variant thereof are the same as described above, and the IL-1 receptor antagonist may have the amino acid sequence of SEQ ID NO: 8. The Fc region may be derived from human IgG4.

Specifically, the fusion protein may be the second fusion protein described above and may consist of the structural formula (V) or (VI).

In another aspect of the present invention, there is provided a fusion protein dimer in which the two fusion proteins are linked. In the present invention, it may exist as a dimer of the two fusion proteins, in which an IL-1 receptor antagonist or a fragment thereof is linked to an Fc region fragment or a variant thereof, are linked. The fusion protein may be linked by cysteine included in the linker located at the N terminus of the Fc region fragment or a variant thereof. At that time, the linker may comprise the hinge region of an immunoglobulin.

In another aspect of the present invention, there is provided a pharmaceutical composition for preventing or treating degenerative brain diseases, comprising a fusion protein comprising an IL-1 receptor antagonist or a fragment thereof; and an Fc region fragment or a variant thereof, or a fusion protein dimer as an active ingredient. The fusion protein, the fusion protein dimer, the degenerative brain disease, the prevention, and the treatment are the same as described above.

A preferred dosage of the pharmaceutical composition may be in the range of 0.01 µg/kg to 10 g/kg per day, or in the range of 0.01 mg/kg to 1 g/kg, depending on the patient's condition, body weight, sex, age, severity of the patient, and route of administration. Such a dosage should not be construed as limiting the scope of the present invention in any aspect.

In another aspect of the present invention, there is provided a use of the pharmaceutical composition comprising a fusion protein comprising an IL-1 receptor antagonist or a fragment thereof; and an IgG4-derived Fc region fragment or a variant thereof, or a fusion protein dimer as an active ingredient for the prevention or treatment of degenerative brain diseases. The IL-1 receptor antagonist or a fragment thereof, the IgG4-derived Fc region fragment or a variant thereof, the fusion protein, the fusion protein dimer, and the pharmaceutical composition are the same as described above.

In another aspect of the present invention, there is provided a use of the pharmaceutical composition comprising a fusion protein comprising an IL-1 receptor antagonist or a fragment thereof; and an IgG4-derived Fc region fragment or a variant thereof, or a fusion protein dimer as an active ingredient for manufacture of a medicament for preventing or treating degenerative brain diseases. The IL-1 receptor antagonist or a fragment thereof, the IgG4-derived Fc region fragment or a variant thereof, the fusion protein, the fusion protein dimer, and the pharmaceutical composition are the same as described above.

In another aspect of the present invention, there is provided a method for preventing or treating degenerative brain diseases, comprising administering the pharmaceutical composition comprising the fusion protein or fusion protein dimer as an active ingredient to a subject. The fusion protein, the fusion protein dimer, the pharmaceutical composition, the administration, the degenerative brain disease, the prevention, and the treatment are the same as described above.

The "subject" may be a mammal, and preferably, may be a human. In addition, the subject may be a patient suffering from a degenerative brain disease or may be a subject with a high probability of suffering from a degenerative brain disease.

Route of administration, dosage, and frequency of administration of the composition may vary depending on the patient's condition and the presence or absence of side effects, and thus the composition may be administered to a subject in various ways and amounts. The optimal administration method, dosage, and frequency of administration can be selected in an appropriate range by those of ordinary skill in the art. In addition, the composition may be administered in combination with any compound or natural extract, which is known to have a therapeutic effect on degenerative brain diseases, or may be formulated in the form of combination preparations with other drugs.

### Mode for Carrying out the Invention

Hereinafter, the present invention will be described in more detail by way of the following examples. However, the following examples are only for illustrating the present invention, and the scope of the present invention is not limited only to these examples.

### Preparation Example 1. Preparation of GLP-1-IgG4 Fc-IL-1Ra fusion protein dimer: GI210B1

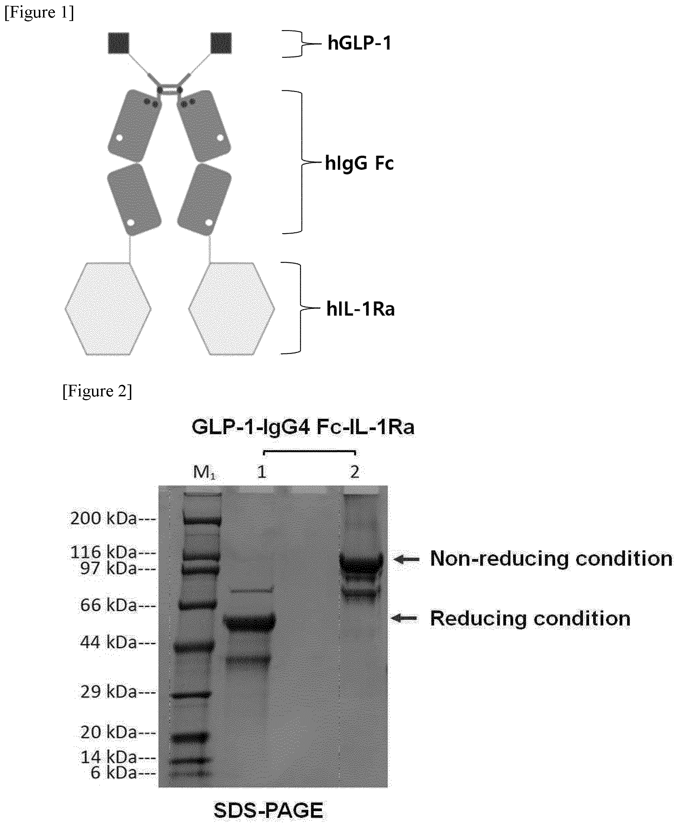

In order to produce a fusion protein dimer comprising GLP-1 (glucagon-like peptide-1), an Fc domain of IgG4 (IgG4 Fc), and an IL-1 receptor antagonist (interleukin-1 receptor antagonist, IL-1Ra), a polynucleotide (SEQ ID NO: 20) encoding a fusion protein (Figure 1) comprising the GLP-1 (SEQ ID NO: 2), the NL028 hinge linker (SEQ ID NO: 5), the IgG4 Fc domain (SEQ ID NO: 6), the linker (SEQ ID NO: 7), and the II,-1Ra (SEQ ID NO: 8) was loaded into the pcDNA3.4 vector (Genscript). The vector was introduced into the CHO cells (ExpiCHO-S cells) to express a fusion protein having the amino acid sequence of SEQ ID NO: 10.

After introducing the vector, the cells were cultured for 14 days using serum-free ExpiCHO^{™} expression medium (Thermo Fisher Scientific) at 37 °C and 8% CO₂ conditions. After culturing, the culture solution was collected, and the fusion protein was purified using affinity chromatography (affinity purification column).

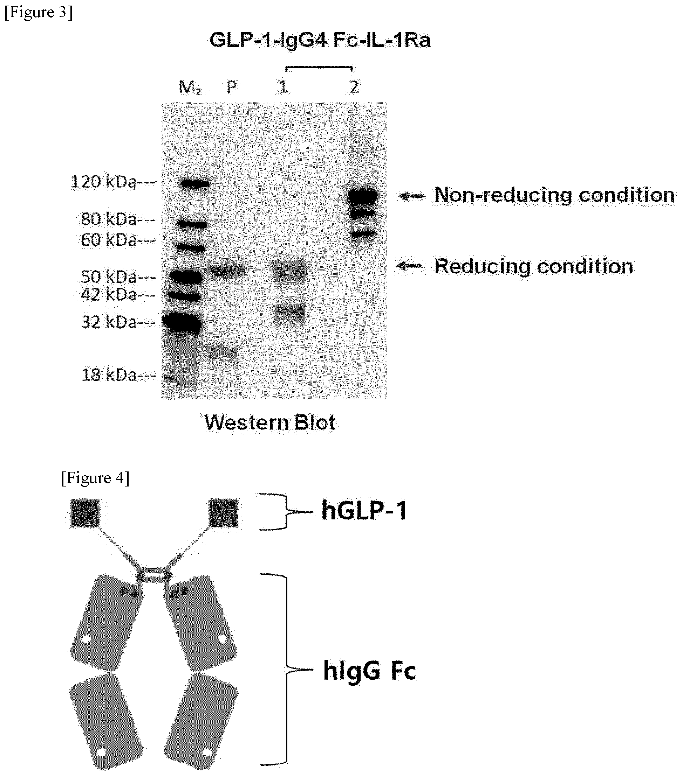

The molecular weight and purity of the purified fusion protein were confirmed by SDS-PAGE and Western blot. As a result of SDS-PAGE and Western blot analysis, it was confirmed that the protein was detected under non-reducing conditions and reducing conditions. Through this, it was confirmed that the purified fusion protein forms a dimer (Figures 2 and 3). The fusion protein dimer was designated as "GI210B1 (also referred to as GLP-1-IgG4 Fc-IL-1Ra)" (Figure 1).

### Preparation Example 2. Preparation of GLP-1-IgG4 Fc fusion protein dimer: GI210B1CN

In order to produce a fusion protein dimer comprising GLP-1 and an Fc domain of IgG4, a polynucleotide (SEQ ID NO: 23) encoding a fusion protein comprising the GLP-1 (SEQ ID NO: 2), the NL028 hinge linker (SEQ ID NO: 5), and the IgG4 Fc domain (SEQ ID NO: 6) was loaded into the pcDNA3.4 vector (Genscript). The vector was introduced into the CHO cells (ExpiCHO-S cells) to express a fusion protein having the amino acid sequence of SEQ ID NO: 22. After introducing the vector, the cells were cultured for 14 days using serum-free ExpiCHO^{™} expression medium (Thermo Fisher Scientific) at 37 °C and 8% CO₂ conditions. After culturing, the culture solution was collected, and the fusion protein was purified using affinity chromatography. The fusion protein dimer was designated as "GI210B1CN (also referred to as GLP-1-IgG4 Fc)" (Figure 4).

### Preparation Example 3. Preparation of IgG4 Fc-IL-1Ra fusion protein dimer: GI210B1C1

In order to produce a fusion protein dimer comprising an IgG4 Fc domain (IgG4 Fc) and an IL-1 receptor antagonist (II,-1Ra), a polynucleotide (SEQ ID NO: 25) encoding a fusion protein comprising the NH06 hinge (SEQ ID NO: 31), the IgG4 Fc domain (SEQ ID NO: 6), the linker (SEQ ID NO: 7), and the II,-1Ra (SEQ ID NO: 8) was loaded into the pcDNA3.4 vector (Genscript). The vector was introduced into the CHO cells (ExpiCHO-S cells) to express a fusion protein having the amino acid sequence of SEQ ID NO: 24.

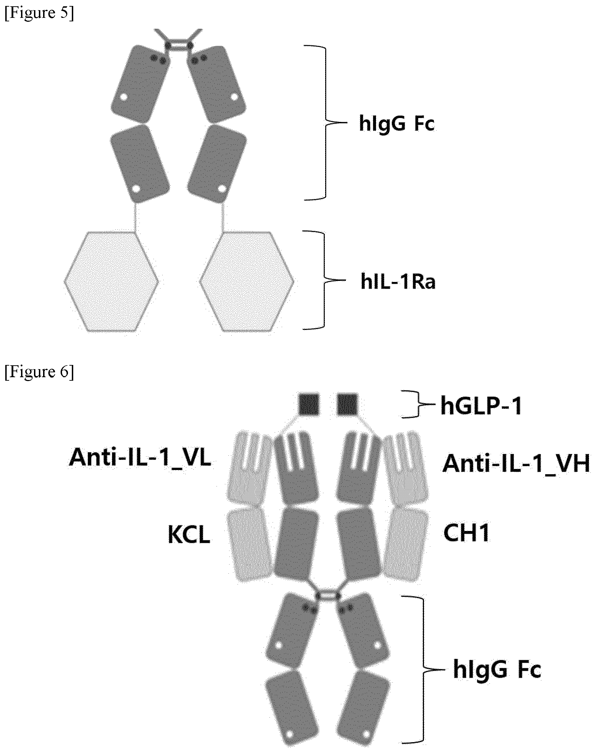

After introducing the vector, the cells were cultured for 14 days using serum-free ExpiCHO^{™} expression medium (Thermo Fisher Scientific) at 37 °C and 8% CO₂ conditions. After culturing, the culture solution was collected, and the fusion protein was purified using affinity chromatography. The fusion protein dimer was designated as "GI210B1C1 (also referred to as IgG4 Fc-II,-1Ra)" (Figure 5).

### Preparation Example 4. Preparation of GLP-1-IgG4 Fc-anti-IL-1 bispecific antibody: GI212B2

In order to produce a bispecific antibody comprising GLP-1 (glucagon-like peptide-1), a fragment of an anti-IL-1 antibody, and an Fc domain of IgG4 (IgG4 Fc), a polynucleotide (SEQ ID NO: 28) encoding the GLP-1 (SEQ ID NO: 2), the (G4S)3 linker (SEQ ID NO: 37), the heavy chain variable region-constant region 1 (CH1) of the anti-IL-1 antibody (SEQ ID NO: 27), the NH06 hinge (SEQ ID NO: 31), and the IgG4 Fc domain (SEQ ID NO: 38), and a polynucleotide (SEQ ID NO: 30) encoding the light chain of the anti-IL-1 antibody (SEQ ID NO: 29) were loaded into the pcDNA3.4 vector (Genscript).

The vector was introduced into the CHO cells (ExpiCHO-S cells) to express a bispecific antibody. After introducing the vector, the cells were cultured for 7 days using serum-free ExpiCHO^{™} expression medium (Thermo Fisher Scientific) at 37 °C and 5% CO₂ conditions. After culturing, the culture solution was collected, and the bispecific antibody was purified using affinity chromatography (affinity purification column).

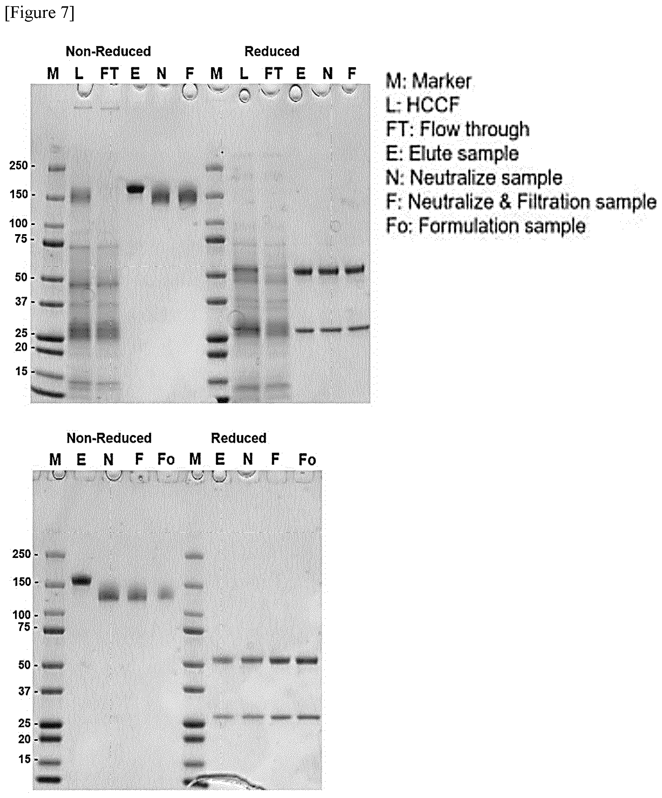

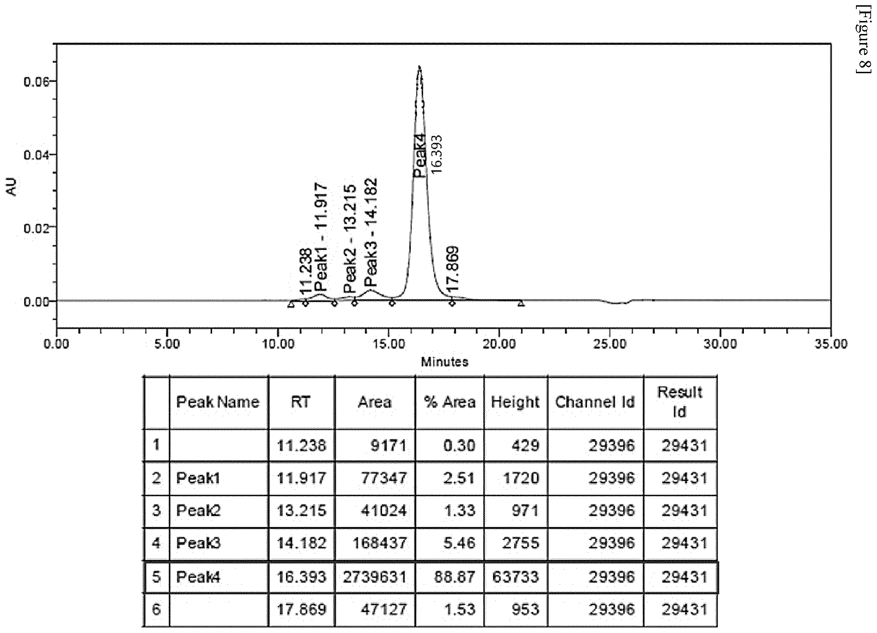

The molecular weight and purity of the purified bispecific antibody were confirmed by SDS-PAGE and SE-HPLC. As a result of SDS-PAGE and SE-HPLC analysis, it was confirmed that the protein was detected under non-reducing conditions and reducing conditions. Through this, it was confirmed that the purified bispecific antibody forms a dimer (Figures 7 and 8). The bispecific antibody was designated as "GI212B2 (also referred to as GLP-1-IgG4 Fc-anti-IL-1 antibody)" (Figure 6).

### Experimental Example 1. Confirmation of binding ability of GI210B1 fusion protein dimer to IL-IRα

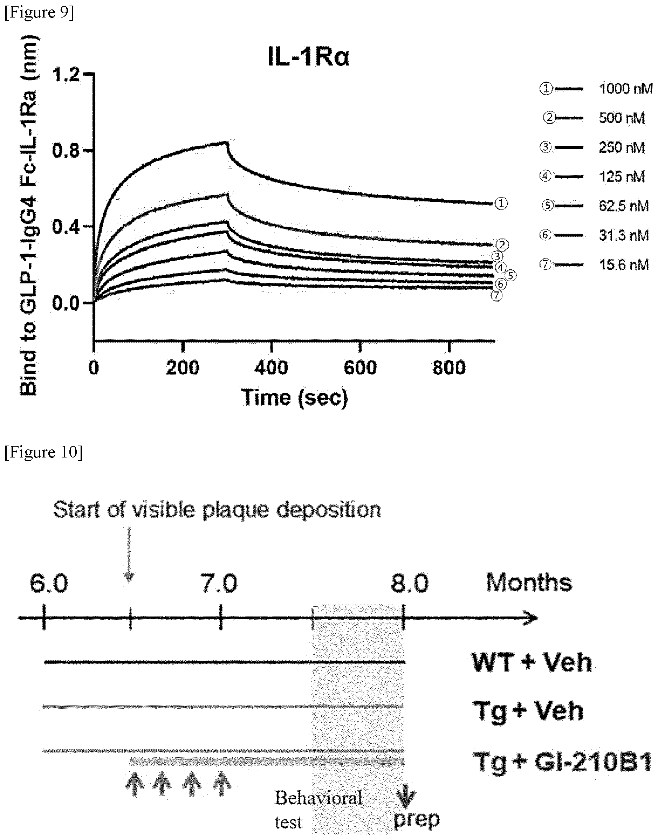

The binding ability of GI210B1, which was obtained through the method of Preparation Example 1 above, to IL-IRα (interleukin-1 receptor alpha) was measured. At that time, the binding ability to IL-IRα was confirmed using Octet RED 384 (ForteBio). The AR2G (amine reactive 2nd generation) biosensor was used by immersion in 1× kinetic buffer for 10 minutes. IL-1Rα (R&D systems) was prepared at a concentration of 1 µg/ml and coated on the biosensor, and the binding ability was confirmed at each concentration by serially diluting GI210B1 from 15.6 nM to 1,000 nM. The binding ability between GI210B1 and IL-IRα is shown in Figure 9 and Table 5 below.

**[Table 5]**

| Kₐ(Association rate, 1/Ms) | K_{d}(dissociation rate, 1/s) | K_{D}(K_{d}/Kₐ, M) |
|---|---|---|
| 2.20E+04 | 9.30E-03 | 423 nM |

### I. Confirmation of therapeutic effect of fusion protein dimer (GI210B1) on degenerative brain diseases

### Example 1. Confirmation of therapeutic effect of GI210B1 using animal model of Alzheimer's disease

The Tg-APP/PS1 mouse, the animal model of Alzheimer's disease used in the experiment, is a known Alzheimer's disease experimental animal model that shows age-dependent plaque deposition and cognitive defects. This model clearly shows amyloid beta plaque deposition and cognitive and behavioral deficits at 7 to 8 months of age.

Accordingly, in order to verify the therapeutic effect of GI210B1, a dimer protein of the present invention, on Alzheimer's disease, a long-term experimental plan of more than 10 months was required

This experiment was planned to treat Tg-APP/PS1 mice with GI210B1 from 6.5 months of age, when Alzheimer's disease (AD)-like pathology appears, and to conduct a behavioral test between 7.5 and 8.0 months of age. After behavioral test evaluation, the experimental animals were sacrificed at 8.0 months of age, and biochemical analysis and histological analysis were performed.

### Example 1.1. Preparation of animal model of Alzheimer's disease and drug administration

The Tg-APPswe/PS1dE9 (Tg-APP/PS1) mice, an animal model of Alzheimer's disease, were prepared by mating the Tg-APP/PS1 mice, which were transduced with APP (amyloid precursor protein) and PS1 (presenilin1) genes, and the C57BL6 × C3H hybrid mice in a designated mating cage in a standard SPF (specific pathogen free) animal room at Ewha Womans University, which is certified as a LMO (living modified organism) processing facility.

The Tg-APP/PS1 mouse is known to exhibit visible plaque deposition starting at 6.5 months of age and to exhibit cognitive defects at approximately 7 months of age or older. Accordingly, The Tg-APP/PS1 mouse can provide face validity, construct validity, and predictive validity in deriving drug candidates for treatment of Alzheimer's disease.

The Tg-APP/PS1 mice were maintained in a standard plastic cage in groups of 2 to 3 mice, and were allowed to eat standard laboratory food and water ad libitum. In addition, the animal room was maintained at a humidity of 50 to 60% and at a temperature of 22 to 23 °C, and was operated under a normal light/dark cycle (lights on at 7 a.m.). Meanwhile, the mortality rate of the Tg-APP/PS1 mice varies depending on gender, and the mortality rate for female Tg-APP/PS1 mice is approximately two times higher than that of male Tg-APP/PS1 mice. Therefore, there is a possibility that female Tg-APP/PS1 mice may show increased sensitivity to the drug.

The test substance was prepared by diluting GI210B1 obtained in Preparation Example 1 in saline, and it was intraperitoneally administered to 6.5-month-old Tg-APP/PS1 mice at a concentration of 1.2 mg/kg a total of 4 times for 2 weeks (Figure 10).

At that time, the wild type mice (WT) were used as the normal group, and the Tg-APP/PS1 mice injected with saline (Tg+Veh) were used as the control group.

### Example 1.2. Behavior evaluation of Tg-APP/PS1 mice following GI210B1 administration

In the Tg-APP/PS1 mice intraperitoneally administered with GI210B1 at 6.5 months of age in Example 1.1 above, behavioral characteristics of cognitive function were evaluated at 7.5 to 8.0 months of age. In order to evaluate the behavioral characteristics of cognitive function, a novel object recognition test (NOR test), a passive avoidance test, and a water maze test were performed. At that time, the three experiments for behavioral evaluation were performed according to a method known in the art.

Meanwhile, evaluation of experimental animal behavior is generally performed by comparing animal behavior with that of control experimental animals. Since animal behavior is easily influenced by the animal's previous experience and handling conditions, repeated measurements of the same behavior for a given animal should be avoided. Accordingly, all experiments were conducted taking into account that animal behavioral tests are influenced by many factors, including the experimenter and circadian rhythms.

All behavior evaluations were recorded with a computerized video-tracking system (SMART; Panlab S.I., Barcelona, Spain) and a webcam recording system (HD Webcam C210, Logitech, Newark, CA, USA). At that time, the background sound in the test room was set to 65 dB of white noise, and all behavioral tests were performed during the photoperiod (9 a.m. to 3 p.m.).

In addition, the test was conducted by limiting the number of animals that could be subjected to a series of behavioral tests for the following reasons: (i) each person can handle up to 50 animals in each behavioral test trial (generally 50 animals per day is too many; behavior is measured for each animal, which requires a set amount of time), (ii) animal behavior is influenced by the experimenter, which requires the same person to collect the series of behavioral tests, and (iii) animal behavior is influenced by circadian rhythms (animal behavior should be collected within a given period of time per day).

Meanwhile, all animals were treated in accordance with the animal care guidelines of Ewha Womans University, and the test was conducted with the approval of the institutional animal care and use committee (IACUC) of Ewha Womans University.

### Example 1.2.1. Confirmation of memory improvement effect of GI210B1 through novel object recognition test

In general, mice have a tendency to prefer novel objects. This is a behavioral characteristic that is possible only when one is able to distinguish between a familiar object and a novel object and remember them when a familiar object and a novel object are presented. Accordingly, object recognition memory can be evaluated through a novel object recognition test as a means of evaluating whether one has a cognitive defect. Cognitive function for a novel object is regulated in areas such as the prefrontal cortex, dorsal striatum, and hippocampus.

Specifically, the mice prepared in Example 1.1 above were repeatedly exposed to two objects of the same shape twice at intervals of 10 minutes in an open field (familiarization). At that time, the mice can distinguish the locations of the objects because the two objects have a distance difference from the external spatial cue. In addition, two hours after the habituation of the mice was completed, the mice were exposed to a novel object and an old object, and then the time taken for the mouse to recognize each object was measured [2-hour-NOR (novel object recognition) experiment]. Thereafter, the mice returned to their home cage, and then the location of one of the just-exposed objects was moved, and then object distinguishment and recognition time were measured [2-hour-NLR (novel position recognition) experiment]. Thereafter, 24 hours after the habituation of the mice was completed, the mice were exposed to another object and an old object, and then the time taken for the mouse to approach each object was measured (24-hour-NOR experiment). The mice recognized the first two objects presented for a similar amount of time without bias regarding the object locations (A in Figure 11).

As a result, in the 2-hour-NOR experiment, it was confirmed that the time taken for the mouse to recognize a novel object was increased in the normal group (WT), while the time taken for the mouse to recognize a novel object was not increased in the control group (Tg+Veh). In the case of the Tg-APP/PS1 mice administered with GI210B1 (Tg+GI210B1) as an experimental group, the time taken for the mouse to recognize a novel object was increased similar to the normal group (WT) (B in Figure 11).

In addition, in the 2-hour-NLR experiment, it was confirmed that the time taken for the mouse to recognize an object whose location was changed was further increased in the normal group (WT), while the time taken for the mouse to recognize an object whose location was changed was not increased in the control group (Tg+Veh). In the case of the Tg-APP/PS1 mice administered with GI210B1 (Tg+GI210B1) as an experimental group, it was confirmed that the time taken for the mouse to recognize an object whose location was changed was increased similar to the normal group (C in Figure 11).

Through the results of the 2-hour-NOR experiment and the 2-hour-NLR experiment, it was confirmed that GI210B1 had an effect of restoring short-term memory lasting for 2 hours for the memory function of the Tg-APP/PS1 mice to remember and identify different objects and the discrimination function for spatial movement.

In addition, in the 24-hour-NOR experiment, it was confirmed that when exposed to an object of a familiar shape and an object of a completely different shape from the previously exposed object, the normal group (WT) recognized the novel object for a longer time, while the control group (Tg+Veh) recognized two different objects for a similar amount of time. On the other hand, it was confirmed that the Tg-APP/PS1 mice administered with GI210B1 (Tg+GI210B1) as an experimental group recognized a novel object for a longer time at a similar level to the normal group (WT) (D in Figure 11). The above results indicate that the long-term memory of the Tg-APP/PS1 mice administered with GI210B1 (Tg+GI210B1) was recovered to the level of being able to distinguish between objects even after 24 hours.

Through the above results, it was confirmed that GI210B1 has a protective effect against short-term and long-term cognitive dysfunction in the Tg-APP/PS1 mice.

### Example 1.2.2. Confirmation of memory improvement effect of GI210B1 through water maze test

The water maze test is a cognitive behavior evaluation experiment conducted to measure spatial perception learning ability and memory dependent on the hippocampus area of the brain. Spatial cues were installed outside the water maze to help mice perceive space, allowing mice to learn and remember the location of the platform below the water based on external cues. The location of the platform inside the water maze is the same, but the starting point for each training is different, so the mouse must clearly learn the water maze and spatial cues and remember the location to find the platform. At that time, since the hidden platform is hidden under the water, its location can be confirmed and identified based on external spatial cues.

Specifically, for spatial perception learning and training of the mice prepared in Example 1.1 above, the mice were trained to visit the hidden platform in the water tank twice a day, in the morning and afternoon, for 5 days. All mice were started from the opposite site of the platform to measure whether they remembered the space where the hidden platform was located.

As a result, it was confirmed that the normal group (WT) began to recognize the surrounding spatial indicators and effectively locate the platform 3 days after starting training to find the hidden platform. On the other hand, it was confirmed that in the control group (Tg+Veh), the time taken for the mouse to find the platform gradually decreased as training progressed, showing a significant difference in the time taken for the mouse to find the platform compared to the wild type mice. Through this, it was confirmed that the spatial perception learning ability of the control group (Tg+Veh) was decreased compared to the normal group (A in Figure 12).

Meanwhile, it was confirmed that in the Tg-APP/PS1 mice administered with GI210B1 (Tg+GI210B1) as an experimental group, the time taken for the mouse to learn and find the location of the platform through spatial clues was somewhat slower than that of the normal group, but faster than that of the control group (Tg+Veh). Through this, it was confirmed that the Tg-APP/PS1 mice administered with GI210B1 (Tg+GI210B1) learned spatial perception more smoothly than the control group (Tg+Veh) (A in Figure 12).

In addition, a hidden platform test was conducted on the 6th day. The hidden platform of the water maze was removed, and then the mouse was placed in the water maze and the location the mouse stayed for 60 seconds was recorded. At that time, the time and location that the mouse stayed in the four quadrants of the water maze for 60 seconds in the absence of a hidden platform were recorded. All mice started from the opposite quadrant of the target where the platform was located.

As a result, it was confirmed that the normal group (WT) stayed in the target quadrant where the platform was located for a longer time than in the other quadrants in the water maze divided into four quadrants. In addition, it was observed searching for the missing platform by hovering around the location where the platform was hidden. In the control group (Tg+Veh), free-swimming behavior was observed continuously without distinguishing between the target quadrant and the non-target quadrant. On the other hand, in the case of the Tg-APP/PS1 mice administered with GI210B1 (Tg+GI210B1) as an experimental group, it was observed that the target quadrant was recognized for a longer time compared to the control group (Tg+Veh) (B in Figure 12).

In addition, in a visual platform test, a flag was placed on the platform so that the mouse could visually determine the location of the platform. As a result, it was observed that mice in all groups found the platform within 60 seconds. Through the above results, it was confirmed that there is no possibility that learning or memory was not achieved due to the inability to recognize spatial cues due to visual problems, and that there were no differences in motivation to avoid water, willpower, swimming ability, and the like (C in Figure 12).

Through the above results, it was confirmed that GI210B1 exhibits an effect of improving the spatial perception learning ability of the Tg-APP/PS1 mice and improving spatial perception memory.

### Example 1.2.3. Confirmation of memory improvement effect of GI210B1 through passive avoidance test

The passive avoidance test is a test to confirm fear/anxiety memory by associating spatial contextual cues and shock in mice for long periods of 24 hours, 72 hours, and 120 hours. The passive avoidance test is a cognitive behavior evaluation experiment conducted to confirm memories associated with spatial and contextual cues, specifically fear and anxiety. Memories associated with fear and anxiety depend on the amygdala, hippocampus, and prefrontal cortex regions of the brain, and are associated with improved learning and memory for fear- and anxiety-inducing stimuli.

Specifically, mice have a tendency to prefer a dark place. Therefore, if a mouse is placed in a light chamber under conditions where it can move randomly in both directions (light chamber-dark chamber), the mouse usually moves to a dark chamber within 10-30 seconds. When a mouse moves to a dark chamber and receives an electric shock, the mouse remembers the relationship between a dark chamber and the electric shock, and when the mouse is placed in a light chamber again, the mouse shows an unwillingness to move to a dark chamber. Using these characteristics, the mouse moved to a dark chamber and then received a foot shock to learn fear/anxiety memory by associating the spatial contextual cue with the shock. The mice prepared in Example 1.1 were learned in the same manner as above, and the time for each step was measured.

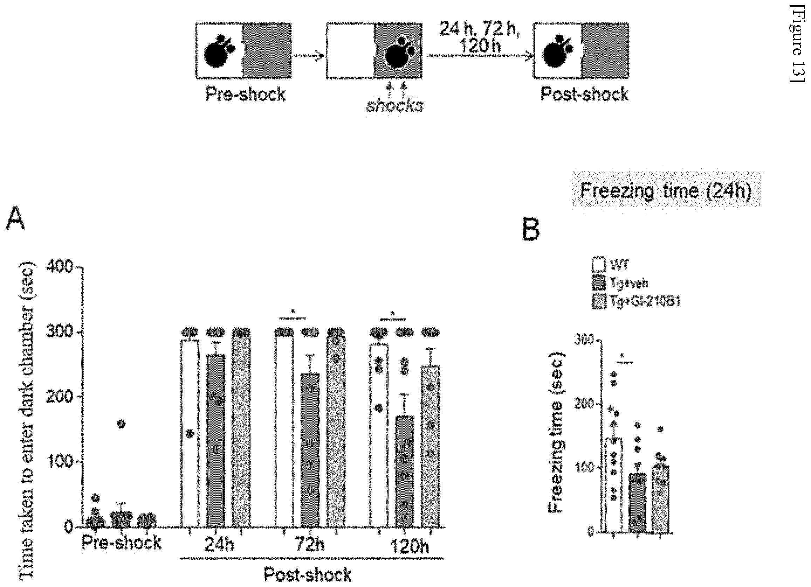

As a result, in the normal group (WT), the mice did not move to a dark chamber for 300 seconds even 24, 72, and 120 hours after the shock was applied to the foot. On the other hand, in the control group (Tg+Veh), the mice began moving to a dark chamber 24 hours after the shock was applied to the foot, and as the memory time became longer, the moving time became faster and the number of moving mice was increased (A in Figure 13). Through this, it was confirmed that the normal group (WT) well maintained the fear memory associated with foot shock and a dark chamber, and that this memory was maintained for a long period of more than 120 hours. On the other hand, it was confirmed that the control group (Tg+Veh) had an impairment in maintaining memories associated with the fear context. It was confirmed that the freezing time measured 24 hours later was shorter in the control group (Tg+Veh) than that of the normal group (WT) (B in Figure 13).

On the other hand, it was confirmed that most of the Tg-APP/PS 1 mice administered with GI210B1 (Tg+GI210B1), an experimental group, had a tendency not to move to the dark chamber even 72 and 120 hours after the shock was applied to the foot similar to the normal group (WT) (A in Figure 13).

Through the above results, it was confirmed that GI210B 1 has the effect of restoring abnormalities in the function of nerve cells constituting the amygdala, hippocampus, and prefrontal cortex regions that are involved in the formation and long-term memory maintenance of fear/anxiety memories expressed by Tg mice.

### Example 1.3. Confirmation of improvement effect on plaque deposition in brain tissue following GI210B1 administration

One of the pathological characteristics of Alzheimer's disease is the deposition of senile plaques. The main component of the senile plaque is an aggregated amyloid beta peptide (Aβ), which causes necrosis of brain nerve cells, leading to the development of Alzheimer's disease.

Accordingly, in order to confirm the effect of treating Alzheimer's disease by intraperitoneal administration of GI210B 1, the Tg-APP/PS 1 mice administered with GI210B1 (Tg+GI210B1) and the Tg-APP/PS1 mice (Tg+Veh), a control group, were sacrificed, and then the brain tissue was extracted, and Aβ plaques were stained using thioflavin S, and then the degree of Aβ plaque deposition in the parietal cortex, hippocampus, and piriform cortex regions was analyzed.

Specifically, for immunohistochemical analysis using thioflavin S staining, the brain was collected after perfusion for the mouse with 4% paraformaldehyde, and then the brain was further fixed with 4% paraformaldehyde overnight at 4 °C. The brain tissue sections were prepared by cutting the fixed brain tissue to a thickness of 40 µm using a vibratome. The prepared brain tissue sections were stained using thioflavin S by a known method. At that time, the stained image was analyzed using MetaMorph Microscopy Automation & Image Analysis software (Molecular device). The amount of plaque was quantified and expressed as the number of plaques per unit area and the sum of the plaque areas.

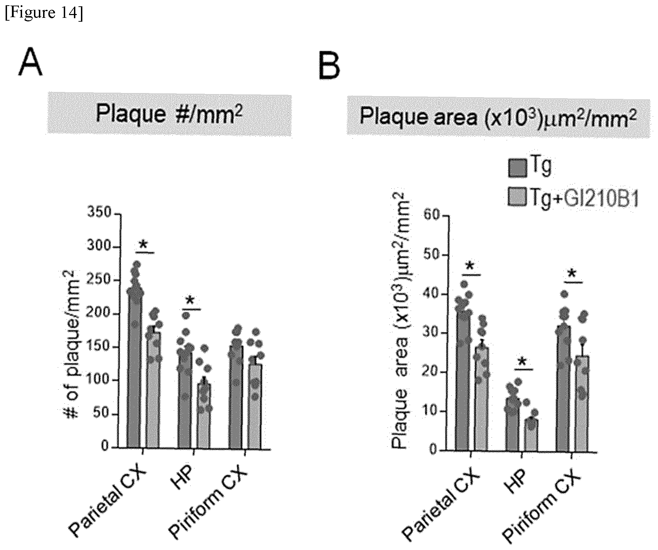

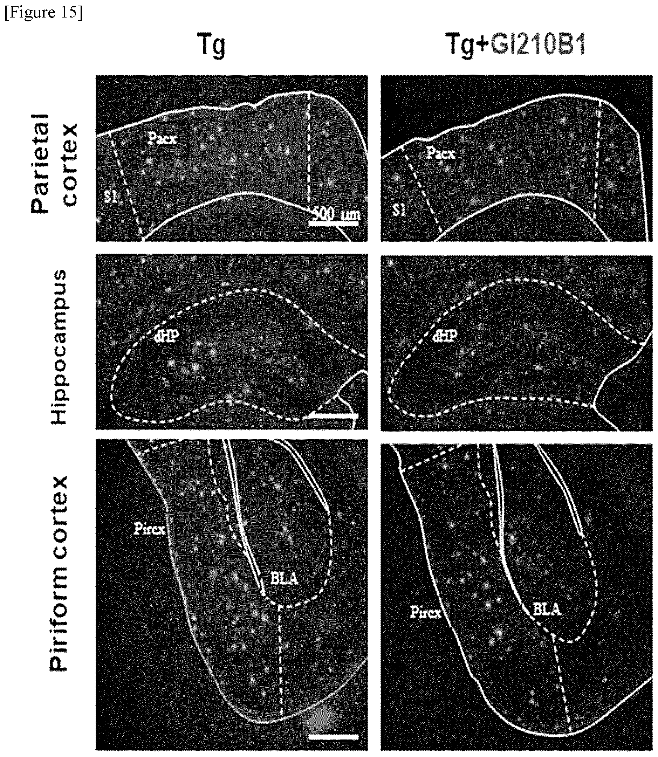

As a result, as shown in Figures 14 and 15, it was confirmed that when GI210B1 was administered, the amount of plaque deposited in the brain of the Tg-APP/PS1 mice tended to be reduced compared to the control group.

Through the above results, it was confirmed that GI210B1 has an effect of reducing Aβ plaque deposition in the brain of the Tg-APP/PS1 mice.

### Example 1.4. Confirmation of neurogenesis and neuronal microstructure protection effects in brain tissue following GI210B1 administration

### Example 1.4.1. Confirmation of neuronal microstructure protection effect through MAP-2 immunofluorescence staining

Long-term memory is regulated at synapses between axons of neurons and dendrites of target neurons, and the microstructure of neurons is greatly influenced by the state of neuron health. Changes in the microstructure of dendrites of neurons are important for maintaining memory. The microstructure of the dendrites of such neurons can be observed as an image by staining with an anti-MAP-2 antibody (GeneTex, Cat No. GTX133109) located in the dendrites.

Therefore, in order to determine the mechanism of action of cognitive function improvement and memory recovery effects of GI210B1, the Tg-APP/PS1 mice administered with GI210B1 (Tg+GI210B1), the wild type mice (WT) as a normal group, and the Tg-APP/PS1 mice (Tg or Tg+Veh) as a control group were sacrificed, and then the brain tissue was extracted, and the microstructural status of the dendrites (dendritic process) of the hippocampus was analyzed.

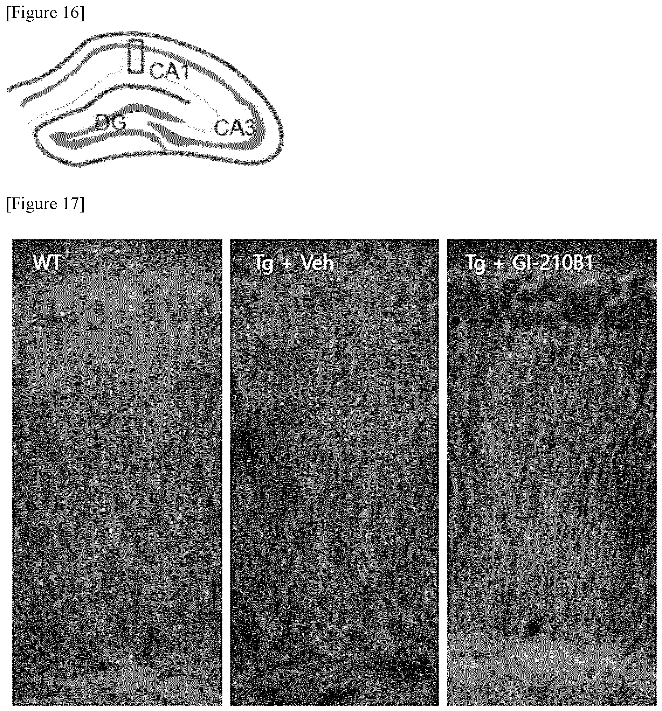

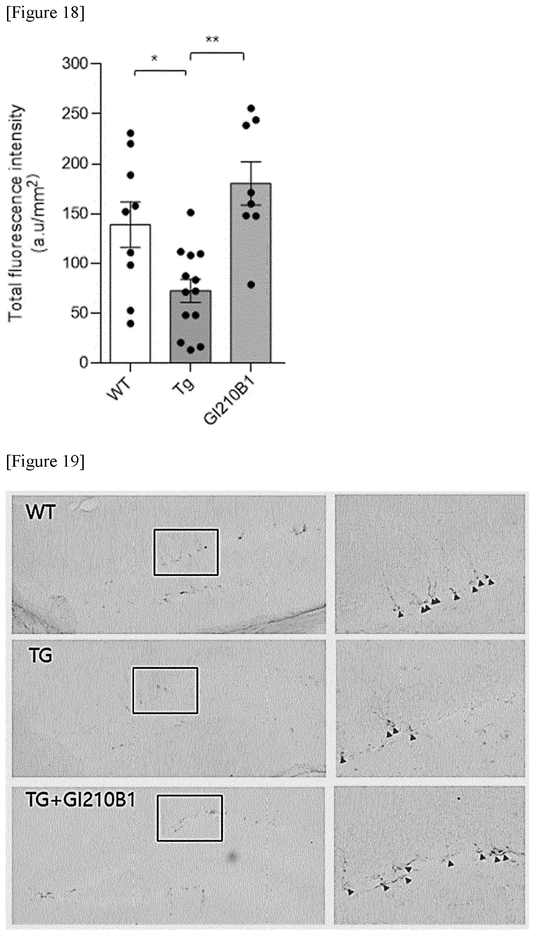

As a result, as shown in Figures 17 and 18, it was confirmed that the expression level of MAP-2 in the CA1 region of the hippocampus (Figure 16) was decreased in the control group (Tg or Tg+Veh) compared to the normal group (WT). On the other hand, it was confirmed that in the case of the Tg-APP/PS1 mice administered with GI210B1 (Tg+GI210B1), the expression level of MAP-2 in the CA1 region of the hippocampus was recovered to the level of the normal group (WT).

Through the above results, it was confirmed that GI210B1 has an effect of protecting the microstructure of dendrites (dendritic process) from reduction/degeneration in the CA1 region of the hippocampus.

### Example 1.4.2. Confirmation of neurogenesis promotion effect through DCX immunochemical staining

DCX (doublecortin) is continuously expressed during the 2-3 weeks during which immature neurons mature, and is expressed in differentiated neurons. For this reason, DCX is used as a marker of neurogenesis, indicating the degree of neuronal differentiation and after the metaphase of newly generated neurons.

In order to confirm the effect of GI210B1 on the decreased neurogenesis in the DG (dentate gyrus) region of the hippocampus of Tg mice, the Tg-APP/PS1 mice administered with GI210B1 (Tg+GI210B 1), the wild type mice (WT) as a normal group, and the Tg-APP/PS1 mice (Tg or Tg+Veh) as a control group were sacrificed, and then the brain tissue was extracted, and the DG region of the hippocampus was stained with DCX.

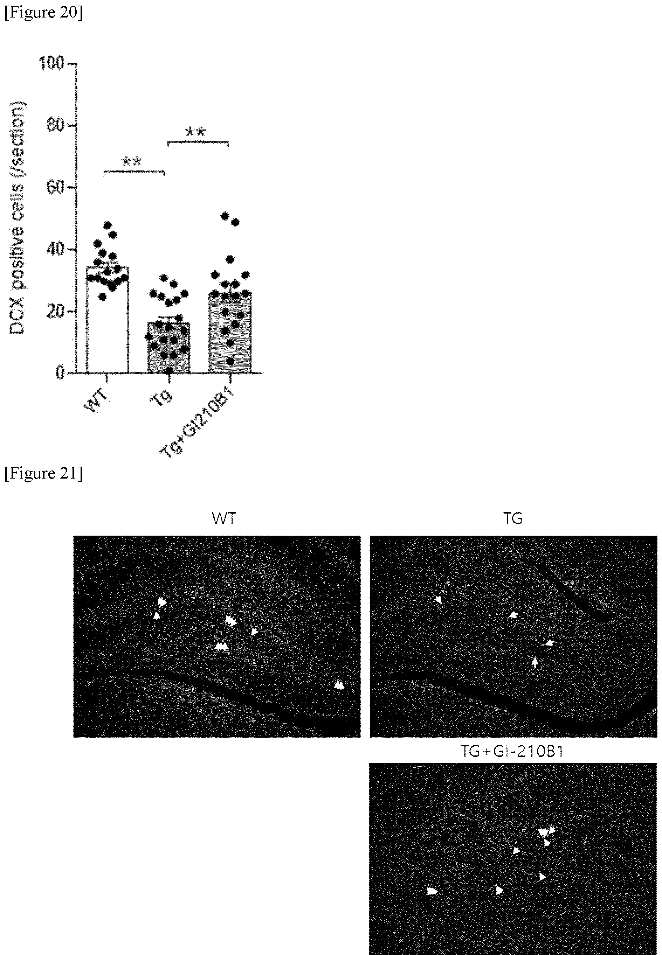

As a result, as shown in Figures 19 and 20, in the case of the control group (Tg), the number of DCX positive cells in the DG region of the hippocampus was significantly reduced compared to the normal group (WT). On the other hand, it was confirmed that in the case of the Tg-APP/PS1 mice administered with GI210B1 (Tg+GI210B1) as an experimental group, the number of DCX positive cells in the DG region of the hippocampus was increased compared to the control group (Tg), and was recovered to a level similar to that of the normal group (WT).

Through the above results, it was confirmed that GI210B1 restores decreased neurogenesis in the DG region of the hippocampus.

### Example 1.4.3. Confirmation of neurogenesis promotion effect through Ki-67 staining

Ki-67 is a protein expressed in the nucleus during cell division. It is expressed throughout cell division (proliferation phase), starting from mid-G1 phase and including S, G2, and M phases, but is not expressed in cells in G0 and early G1 phases where cell division does not occur.

In order to confirm the effect of GI210B1 on the decreased neurogenesis in the DG region of the hippocampus of Tg mice, the Tg-APP/PS1 mice administered with GI210B1 (Tg+GI210B 1), the wild type mice (WT) as a normal group, and the Tg-APP/PS1 mice (Tg) as a control group were sacrificed, and then the brain was extracted, and the DG region of the hippocampus was fluorescently stained with Ki-67.

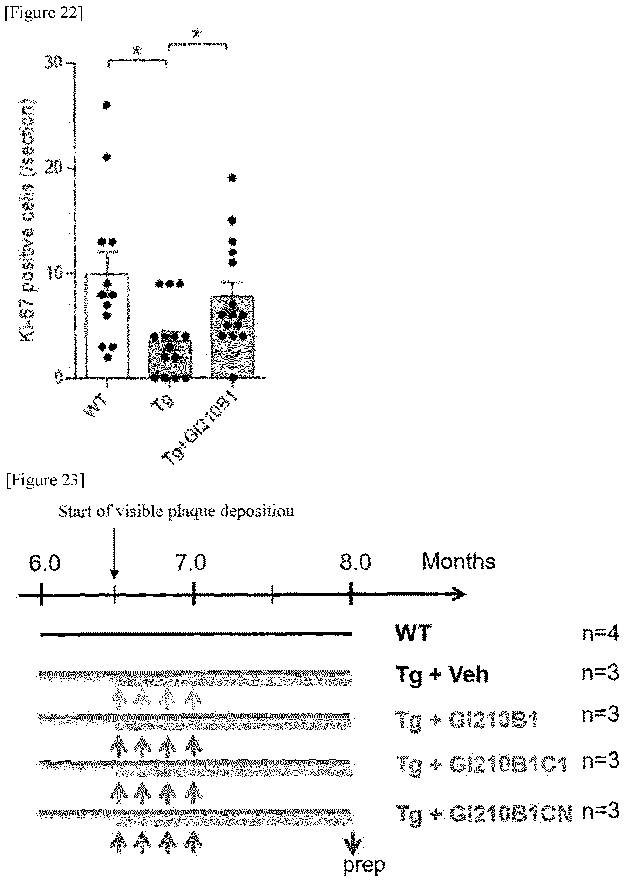

As a result, as shown in Figures 21 and 22, in the case of the control group (Tg), the number of Ki-67 positive cells in the DG region of the hippocampus was significantly reduced compared to the normal group (WT). On the other hand, in the case of the Tg-APP/PS1 mice administered with GI210B1 (Tg+GI210B1), the number of Ki-67 positive cells was increased compared to the Tg-APP/PS1 mice (Tg), which was a level similar to that of the normal group (WT).

Through the above results, it was confirmed that GI210B1 restores decreased neurogenesis in the DG region of the hippocampus.

### II. Confirmation of therapeutic effect of fusion protein dimer on degenerative brain diseases

### Example 2. Confirmation of therapeutic effect of fusion protein dimer using animal model of Alzheimer's disease: GI210B1, GI210B1C1, and GI210B1CN

The Tg-APP/PS1 mice were prepared in the same manner as in Example 1.1 above. The test substance was prepared by diluting GI210B1, GI210B1C1, or GI210B1 CN obtained in Preparation Example 1 to 3 in saline. Thereafter, the prepared test substances were each intraperitoneally administered to the 6.5-month-old Tg-APP/PS1 mice at a concentration of 1.2 mg/kg, 0.7778 mg/kg, and 1.1745 mg/kg a total of 4 times for 2 weeks (Figure 23).

At that time, the wild type mice administered with PBS (WT+Veh) were used as the normal group, and the Tg-APP/PS1 mice administered with PBS (Tg+Veh) were used as the control group.

### Example 2.1. Confirmation of improvement effect on plaque deposition in brain tissue through thioflavin S staining

In the same manner as in Example 1.3 above, the mice were sacrificed, and then the brain tissue was extracted, and the degree of Aβ plaque deposition in the parietal cortex, hippocampus, and piriform cortex regions was analyzed using thioflavin S, which stains Aβ plaques.

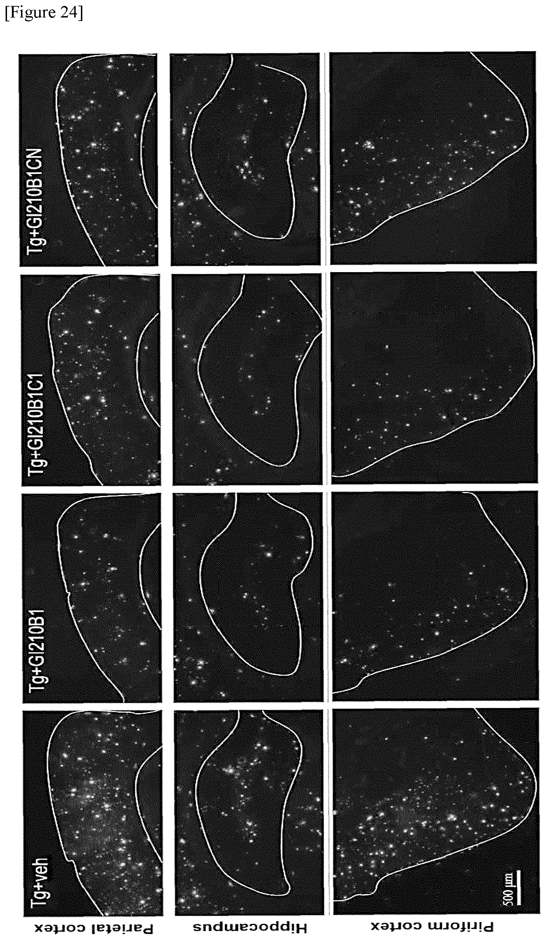

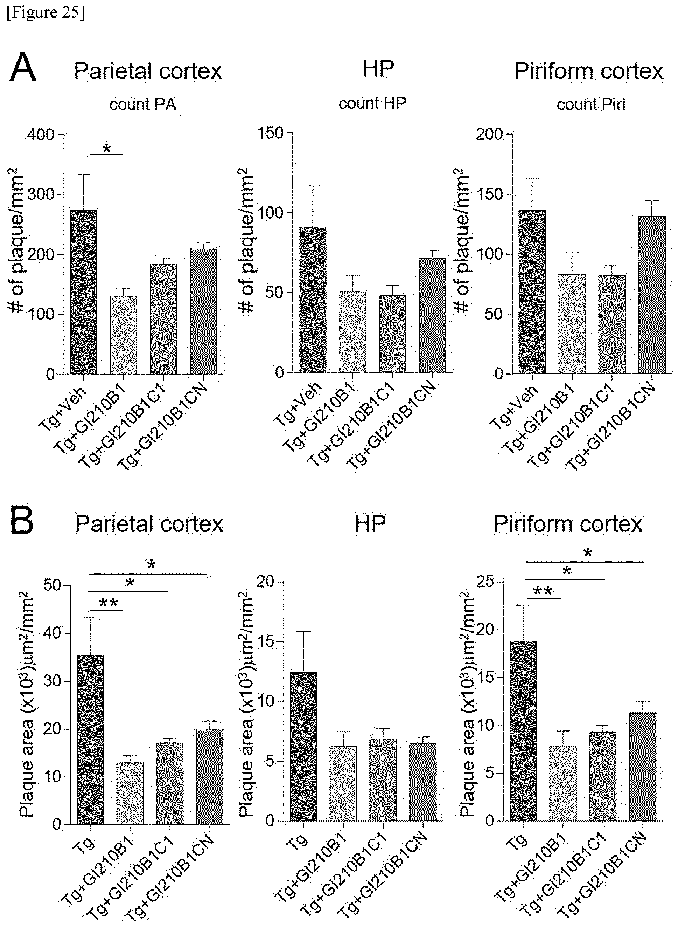

As a result, as shown in Figures 24 and 25, it was confirmed that the amount of plaque deposited in the brain of the Tg-APP/PS1 mice administered with GI210B1, GI210B1C1, or GI210B1CN tended to be reduced. In particular, it was confirmed that when GI210B1 or GI210B1C1 was administered, the amount of plaque deposited in the brain was statistically significantly reduced compared to the control group (Tg+Veh). At that time, The amount of plaque was quantified and expressed as the number of plaques per unit area and the sum of the plaque areas.

Through the above results, it was confirmed again that GI210B1 and GI210B1C1 have an effect of reducing Aβ plaque deposition in the brain of the Tg-APP/PS1 mice.

### Example 2.2. Confirmation of neurogenesis and neuronal microstructure protection effects in brain tissue following GI210B1C1 administration

### Example 2.2.1. Confirmation of neuronal microstructure protection effect through MAP-2 immunofluorescence staining

In the same manner as in Example 1.4.1 above, the mice from each group were sacrificed, and then the brain tissue was extracted, and the microstructural status of the dendrites of the hippocampus was analyzed.

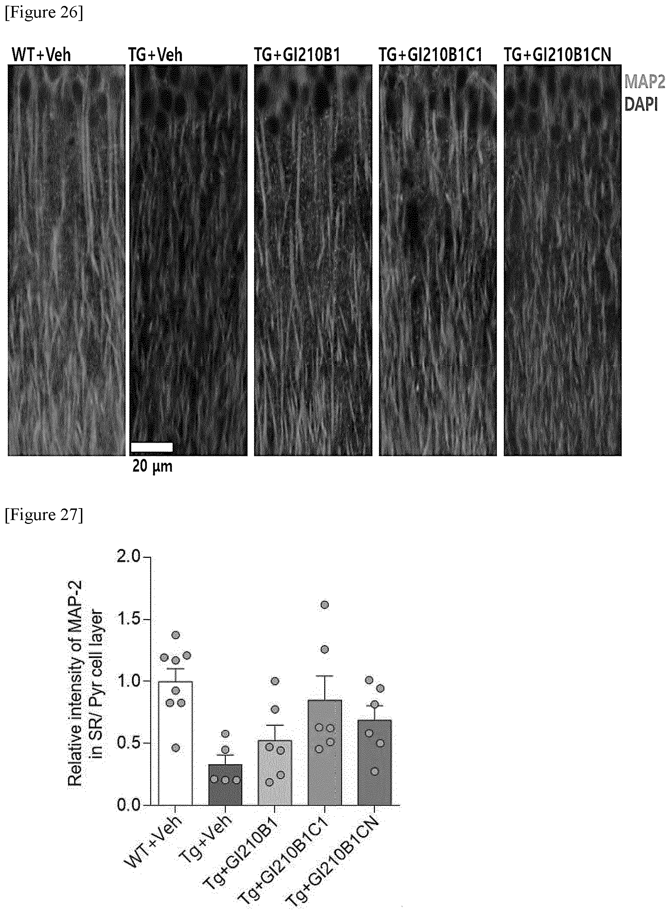

As a result, as shown in Figures 26 and 27, it was confirmed that in the case of the control group (Tg+Veh), the expression level of MAP-2 in the CA1 region of the hippocampus was significantly reduced compared to the normal group (WT+Veh). On the other hand, it was confirmed that in the case of the Tg-APP/PS1 mice administered with GI210B1, GI210B1C1, or GI210B1CN, the expression level of MAP-2 in the CA1 region of the hippocampus was higher than that of the control group (Tg+Veh). In particular, it was confirmed that the expression level of the mice administered with GI210B1C1 was recovered to a level similar to that of the normal group.

Through the above results, it was confirmed that GI210B1C1 has an effect of protecting the microstructure of dendrites (dendritic process) from reduction/degeneration in the CA1 region of the hippocampus.

### Example 2.2.2. Confirmation of neurogenesis promotion effect through DCX immunochemical staining

In the same manner as in Example 1.4.2 above, the mice from each group were sacrificed, and then the brain tissue was extracted, and the DG region of the hippocampus was stained with DCX.

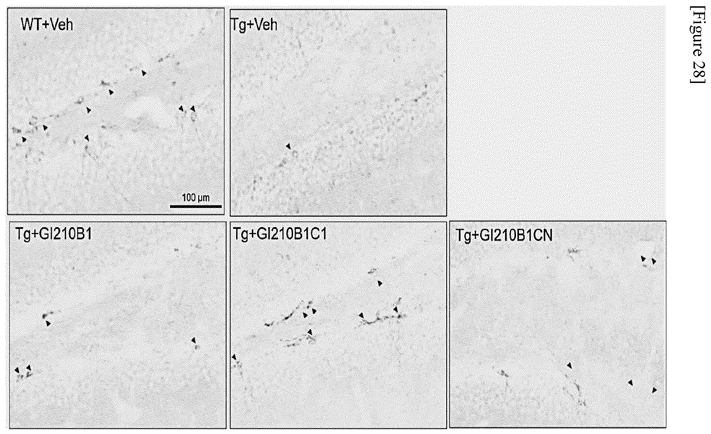

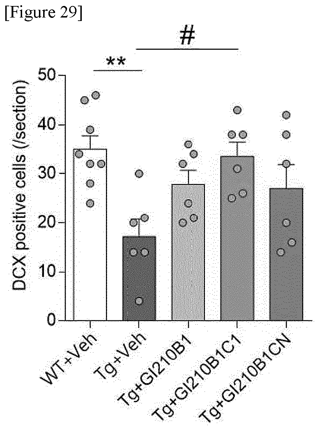

As a result, as shown in Figures 28 and 29, in the case of the control group (Tg+Veh), the number of DCX positive cells in the DG region of the hippocampus was significantly reduced compared to the normal group (WT+Veh). On the other hand, it was confirmed that in the case of the Tg-APP/PS1 mice administered with GI210B1C1 (Tg+GI210B1C1), the number of DCX positive cells in the DG region of the hippocampus was statistically significantly increased compared to the control group (Tg+Veh), and was recovered to a level similar to that of the normal group (WT+Veh).

Through the above results, it was confirmed that GI210B1C1 restores decreased neurogenesis in the DG region of the hippocampus.

### Example 2.3. Measurement of body weight

In order to confirm the change in body weight due to drug administration, the body weight of the mice was measured during the drug administration period and before the mice are sacrificed.

As a result, it was confirmed that there was no significant change in body weight in all groups compared to the control group.

### III. Confirmation of therapeutic effect of administration of interleukin-1 receptor antagonist and GLP-1 in combination on degenerative brain diseases

### Example 3. Confirmation of therapeutic effect of administration of interleukin-1 receptor antagonist and GLP-1 in combination using animal model of Alzheimer's disease

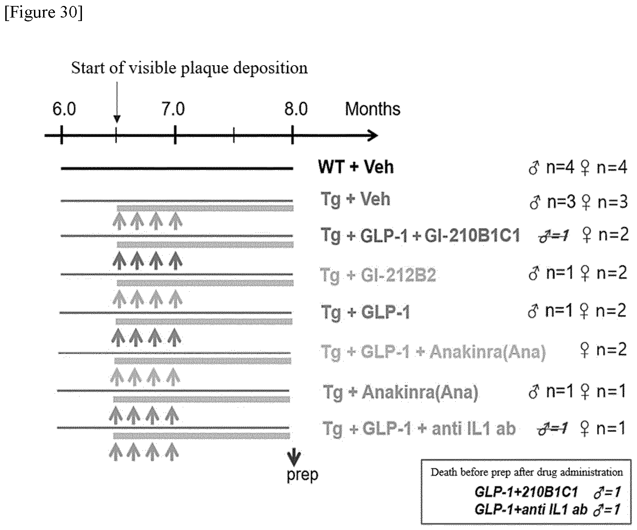

The Tg-APP/PS1 mice were prepared in the same manner as in Example 1.1 above. The test substance was prepared by diluting GLP-1 (Peptron), GI210B1C1 obtained in Preparation Example 2 and GI212B2 obtained in Preparation Example 4, anakinra (Amgen, Kineret-inj), and the anti-IL-1 antibody (BioXcell, BE0246) in saline to concentrations of 0.0371 mg/kg, 1.1745 mg/kg, 1.8722 mg/kg, 0.2041 mg/kg, and 1.77 mg/kg, respectively. Thereafter, the prepared test substances were intraperitoneally administered to the 6.5-month-old Tg-APP/PS1 mice a total of 4 times for 2 weeks, as shown in Table 6 below (Figure 30). At that time, the wild type mice administered with PBS (WT+Veh) were used as the normal group, and the Tg-APP/PS1 mice administered with PBS (Tg+Veh) were used as the control group.

**[Table 6]**

| | Group | Number of subjects (male/female) |
|---|---|---|
| 1 | WT+Veh(PBS) | n=8 (4/4) |
| 2 | Tg+Veh(PBS) | n=6 (3/3) |
| 3 | Tg+GLP-1+GI210B1C1 | n=3 (1/2) male death |
| 4 | Tg+GI212B2 | n=3 (1/2) |
| 5 | Tg+GLP-1 | n=3 (1/2) |
| 6 | Tg+GLP-1+Anakinra(Ana) | n=2 (0/2) |
| 7 | Tg+Anakinra(Ana) | n=2 (1/1) |
| 8 | Tg+GLP-1+anti-IL-1 antibody | n=2 (1/1) male death |

### Example 3.1. Confirmation of improvement effect on plaque deposition in brain tissue following co-administration

In the same manner as in Example 1.3 above, the mice from each group were sacrificed, and then the brain tissue was extracted, and the degree of Aβ plaque deposition in the parietal cortex, hippocampus, and piriform cortex regions was analyzed using thioflavin S, which stains Aβ plaques.

As a result, as shown in Figures 31 to 32c, it was confirmed that the amount of plaque deposited in all of cortex, hippocampus, and piriform cortex of the brain of the Tg-APP/PS1 mice administered with GLP-1 and GI210B1C1 in combination (Tg+GLP-1+GI210B1C1) and the Tg-APP/PS1 mice administered with GI212B2 (Tg+GI212B2) tended to be significantly reduced compared to the control group.

### Example 3.2. Confirmation of neurogenesis and neuronal microstructure protection effects in brain tissue following co-administration

### Example 3.2.1. Confirmation of neuronal microstructure protection effect through MAP-2 immunofluorescence staining

In the same manner as in Example 1.4.1 above, the mice from each group were sacrificed, and then the brain tissue was extracted, and the microstructural status of the dendrites of the hippocampus was analyzed.

As a result, as shown in Figures 33 and 34, it was confirmed that in the case of the control group (Tg+Veh), the expression level of MAP-2 in the CA1 region of the hippocampus was significantly reduced compared to the normal group (WT+Veh). On the other hand, it was confirmed that in the case of the Tg-APP/PS1 mice administered with GLP-1 and GI210B1C1 in combination (Tg+GLP-1+GI210B1C1), the Tg-APP/PS1 mice administered with GLP-1 and anakinra in combination (Tg+GLP-1+Ana), and the Tg-APP/PS1 mice administered with GI212B2 (Tg+GI212B2), the expression level of MAP-2 in the CA1 region of the hippocampus was higher than that of the control group (Tg+Veh), and it was confirmed that the expression level was recovered to a level similar to that of the normal group.

### Example 3.2.2. Confirmation of neurogenesis promotion effect through DCX immunochemical staining

In the same manner as in Example 1.4.2 above, the mice from each group were sacrificed, and then the brain tissue was extracted, and the DG region of the hippocampus was stained with DCX.

As a result, as shown in Figures 35 and 36, in the case of the control group (Tg+Veh), the number of DCX positive cells in the DG region of the hippocampus was significantly reduced compared to the normal group (WT+Veh). On the other hand, it was confirmed that in the case of the Tg-APP/PS1 mice administered with GLP-1 and GI210B1C1 in combination (Tg+GLP-1+GI210B1C1), the Tg-APP/PS1 mice administered with GLP-1 and anakinra in combination (Tg+GLP-1+Ana), and the Tg-APP/PS1 mice administered with GLP-1 and an anti-IL-1 antibody in combination (Tg+GLP-1+anti-IL-1 antibody), the number of DCX positive cells in the DG region of the hippocampus was statistically significantly increased compared to the control group (Tg+Veh), and was recovered to a level similar to that of the normal group (WT+Veh).

At that time, it was confirmed that in the case of the Tg-APP/PS1 mice administered with GLP-1 and anakinra in combination (Tg+GLP-1+Ana), the value was measured high in one mouse, but except for this, the value of the remaining mice was recovered to a level similar to that of the normal group.

### Example 3.3. Measurement of body weight

In order to confirm the change in body weight due to drug administration, the body weight of the mice was measured during the drug administration period and before the mice are sacrificed.

As a result, it was confirmed that there was no significant change in body weight in all groups compared to the control group.

### Sequence listing

1. SEQ ID NO: : 43
Sequence type: amino acid
Sequence name: LCDR2 of Anti-IL-1
Sequence : YAS

## Claims

1. A pharmaceutical composition for preventing or treating degenerative brain diseases, comprising GLP-1 or a variant thereof, and an IL-1 receptor antagonist (interleukin-1 receptor antagonist) or a fragment thereof as active ingredients.

2. The pharmaceutical composition for preventing or treating degenerative brain diseases according to claim 1, wherein the GLP-1 has the amino acid sequence of SEQ ID NO: 1.

3. The pharmaceutical composition for preventing or treating degenerative brain diseases according to claim 1, wherein the variant of GLP-1 has any one of the amino acid sequences of SEQ ID NOs: 2 to 4.

4. The pharmaceutical composition for preventing or treating degenerative brain diseases according to claim 1, wherein the IL-1 receptor antagonist has the amino acid sequence of SEQ ID NO: 8.

5. The pharmaceutical composition for preventing or treating degenerative brain diseases according to claim 1, wherein the IL-1 receptor antagonist or a fragment thereof is a second fusion protein dimer further comprising an Fc region.

6. The pharmaceutical composition for preventing or treating degenerative brain diseases according to claim 5, wherein the second fusion protein comprising an IL-1 receptor antagonist or a fragment thereof, and an Fc region consists of the following structural formula (V) or (VI):
N'-[linker (6)]o-Fc region fragment or variant thereof-[linker (7)]p-B-C' (V)
N'-B-[linker (8)]q-Fc region fragment or variant thereof-C' (VI)
wherein, in the structural formulas (V) and (VI),
N' is the N terminus of the fusion protein,
C' is the C terminus of the fusion protein,
B is an IL-1 receptor antagonist or a fragment thereof,
the linker (6), linker (7), and linker (8) are peptide linkers, and
o, p, and q are each independently 0 or 1.

7. The pharmaceutical composition for preventing or treating degenerative brain diseases according to claim 1, wherein the GLP-1 or a variant thereof, and the IL-1 receptor antagonist or a fragment thereof are a third fusion protein dimer in a fused form.

8. The pharmaceutical composition for preventing or treating degenerative brain diseases according to claim 7, wherein the third fusion protein consists of the following structural formula (I) or (II):
N'-X-[linker (1)]r-Fc region fragment or variant thereof-[linker (2)]s-Y-C' (I)
N'-Y-[linker (1)]r-Fc region fragment or variant thereof-[linker (2)]s-X-C' (II)
wherein, in the structural formulas (I) and (II),
N' is the N terminus of the fusion protein,
C' is the C terminus of the fusion protein,
X is GLP-1 or a variant thereof,
Y is an IL-1 receptor antagonist or a fragment thereof,
the linker (1) and linker (2) are peptide linkers, and
r and s are each independently 0 or 1.

9. The pharmaceutical composition for preventing or treating degenerative brain diseases according to claim 5 or 8, wherein the Fc region is derived from human IgG4.

10. The pharmaceutical composition for preventing or treating degenerative brain diseases according to claim 1, wherein the degenerative brain disease is selected from the group consisting of Alzheimer's disease, Parkinson's disease, Huntington's disease, mild cognitive impairment, cerebral amyloid angiopathy, Down syndrome, amyloid stroke, systemic amyloid disease, Dutch amyloidosis, Niemann-Pick disease, senile dementia, amyotrophic lateral sclerosis, spinocerebellar atrophy, Tourette's syndrome, Friedreich's ataxia, Machado-Joseph's disease, lewy body dementia, dystonia, progressive supranuclear palsy, and frontotemporal dementia.

11. A bispecific antibody in which GLP-1 or a variant thereof is linked to an anti-IL-1 antibody or a fragment thereof.

12. The bispecific antibody according to claim 11, wherein the anti-IL-1 antibody comprises
a heavy chain variable region comprising HCDR1 of SEQ ID NO: 39, HCDR2 of SEQ ID NO: 40, and HCDR3 of SEQ ID NO: 41; and
a light chain variable region comprising LCDR1 of SEQ ID NO: 42, LCDR2 of SEQ ID NO: 43, and LCDR3 of SEQ ID NO: 44.

13. The bispecific antibody according to claim 11, wherein the anti-IL-1 antibody comprises a heavy chain variable region (VH) comprising the amino acid sequence of SEQ ID NO: 32 and a light chain variable region (VL) comprising the amino acid sequence of SEQ ID NO: 34.

14. The bispecific antibody according to claim 11, wherein the anti-IL-1 antibody or a fragment thereof, and the GLP-1 or a variant thereof are linked through a linker.

15. The bispecific antibody according to claim 14, wherein the linker comprises the amino acid sequence of SEQ ID NO: 26.

16. The bispecific antibody according to claim 11, wherein the bispecific antibody consists of the following structural formulas (VII) and (VIII):
N'-[D]t-[linker (9)]u-E-[linker (10)]v-Fc region fragment or variant thereof-[linker (9)]w-[D]x-C' (VII)
N'-[D]y-[linker (9)]z-E'-[linker (9)]a-[D]x-C' (VIII)
wherein, in the structural formulas (VII) and (VIII),
N' is the N terminus of the bispecific antibody,
C' is the C terminus of the bispecific antibody,
D is GLP-1 or a variant thereof,
E is the antigen binding site of the heavy chain of the anti-IL-1 antibody and comprises a variable region (VH) and a CH1 region,
E' is the antigen binding site of the light chain of the anti-IL-1 antibody and comprises a variable region (VL) and a constant region (CL),
the linker (9) and linker (10) are peptide linkers, and
t, u, v, w, x, y, z, and a are each independently 0 or 1.

17. The bispecific antibody according to claim 16, wherein the Fc region is derived from human IgG4.

18. A pharmaceutical composition for preventing or treating degenerative brain diseases, comprising the bispecific antibody in which GLP-1 or a variant thereof is linked to an anti-IL-1 antibody or a fragment thereof, according to claim 11, as an active ingredient.

19. The pharmaceutical composition for preventing or treating degenerative brain diseases according to claim 18, wherein the degenerative brain disease is selected from the group consisting of Alzheimer's disease, Parkinson's disease, Huntington's disease, mild cognitive impairment, cerebral amyloid angiopathy, Down syndrome, amyloid stroke, systemic amyloid disease, Dutch amyloidosis, Niemann-Pick disease, senile dementia, amyotrophic lateral sclerosis, spinocerebellar atrophy, Tourette's syndrome, Friedreich's ataxia, Machado-Joseph's disease, lewy body dementia, dystonia, progressive supranuclear palsy, and frontotemporal dementia.

20. A fusion protein comprising an IL-1 receptor antagonist or a fragment thereof and an Fc region fragment or a variant.

21. The fusion protein according to claim 20, wherein the IL-1 receptor antagonist has the amino acid sequence of SEQ ID NO: 8.

22. The fusion protein according to claim 20, wherein the Fc region is derived from human IgG4.

23. A fusion protein dimer in which the two fusion proteins according to any one of claims 20 to 22 are linked.

24. A pharmaceutical composition for preventing or treating degenerative brain diseases, comprising the fusion protein comprising an IL-1 receptor antagonist or a fragment thereof and an Fc region fragment or a variant, according to claim 20, or the fusion protein dimer according to claim 23 as an active ingredient.

25. A method for preventing or treating degenerative brain diseases, comprising administering the composition according to claim 1, the bispecific antibody according to claim 11, the fusion protein according to claim 20, or the fusion protein dimer according to claim 23 to a subject.

26. A use of the composition according to claim 1, the bispecific antibody according to claim 11, the fusion protein according to claim 20, or the fusion protein dimer according to claim 23 for the prevention or treatment of degenerative brain diseases.

27. A use of the composition according to claim 1, the bispecific antibody according to claim 11, the fusion protein according to claim 20, or the fusion protein dimer according to claim 23 for manufacture of a medicament for preventing or treating degenerative brain diseases.
